# EUROPEAN PATENT SPECIFICATION

(11) **EP 0 343 961 B1**
(45) Date of publication and mention of the grant of the patent: **10.01.1996**
(21) Application number: 89305255.5
(22) Date of filing: 24.05.1989
(51) Int. Cl.: C07D 295/12, A61K 31/495, C07D 239/42, A61K 31/505, C07D 407/12, C07D 209/42, A61K 31/40, C07D 307/84, A61K 31/34

(54) **Aryl- and heteroaryl piperazinyl carboxamides having central nervous system activity**
Aryl- und Heteroaryl-piperazinylcarboxamide mit Wirkung auf das zentrale Nervensystem
Aryl- et hétéroaryl pipérazinyl carboxamides avec activité sur le système nerveux central

(30) Priority: 24.05.1988 US 197890; 13.01.1989 US 297460
(43) Date of publication of application: 29.11.1989
(73) Proprietor: AMERICAN HOME PRODUCTS CORPORATION, Madison, New Jersey 07940-0874 (US)
(72) Inventor: Abou-Gharbia, Magid Abdel-Megid, Glen Mills Pennsylvania 19342 (US); Yardley, John Patrick, Gulph Mills Pennsylvania 19406 (US); Childers, Wayne Everett, Jr., Yardley Pennsylvania 19067 (US)
(74) Representative: Wileman, David Francis, Dr.

(56) References cited:
- EP-A- 0 116 360
- EP-A- 0 138 280
- US-A- 3 646 047
- US-A- 3 734 915
- US-A- 4 001 223

## Description

This invention relates to novel carboxamides, more particularly aryl- and heteroaryl piperazinyl carboxamides having central nervous system activity, methods for making them and pharmaceutical compositions containing them.

The recent introduction of buspirone having a selectivity for 5-HT_{1A} receptors, as an effective anxiolytic agent (United States Patent 3,717,634), into the United States marketplace has stimulated interest in development of second-generation anxiolytic agents.

Furthermore, in clinical trials, gepirone and ipsapirone were found to be potent anxiolytic drugs. Since both drugs -- gepirone and ipsapirone -- possess a higher degree of selectivity for 5-HT_{1A} receptors than buspirone, the clinical data support the notion that anxiety mechanisms can directly be modulated by 5-HT_{1A} receptor drug interactions.

In addition to treatment of anxiety, 5-HT_{1A} agonists such as gepirone are now being examined for their mixed activity as anxiolytic antidepressant agents. The therapeutic potential of 5-HT_{1A} agonists in the treatment of multi-CNS disorders was recently extended to the development of antipsychotic anxiolytic agents represented by MDL-72832 and KC-9172 (Br. J. Pharmocol., 90, 273P, 1987), the latter being under development as an antipsychotic agent (Scrip No. 1265, December 11, 1987). This class of compounds demonstrated high affinity for both the 5-HT_{1A} and D₂ receptor binding sites.

U.S. Patent 4,202,898 describes arylpiperazines useful for the treatment of anxiety and depression. U.S. Patent 4,001,223 describes the synthesis of adamantane derivatives useful as cerebral vasodilators. Abou-Gharbia et al, U.S.S.N. 127,740, filed December 2, 1987, describes the synthesis of adamantyl and fluorenylarylpiperazines with potential CNS activity.

US Patent 4,202,898 discloses synthesis of arylpiperazines of the general formula 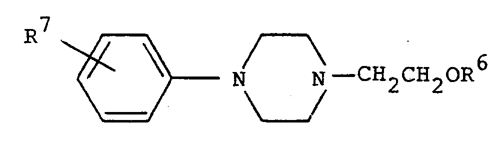
wherein R⁶ is H, CO(lower alkyl), CO(monocyclic aryl), CONH(lower alkyl), CON(lower alkyl) or CONH(monocyclic aryl); R⁷ is H, alkyl, alkoxy, CN, halo or trifluoromethyl useful for the treatment of anxiety and depression.

Compounds of the present invention differ in having a substituted adamantyl, noradamantyl, indolyl or benzofuryl amide moiety attached to the alkylarylpiperazinyl functionality.

US Patent Nos 3646047 and 3734915 disclose benzo[b]furan-2-carboxamide derivatives stated to be useful as tranquillizers, CNS depressants, hypnotics and muscle relaxants.

The present invention provides use of carboxamides characterized by the general formula 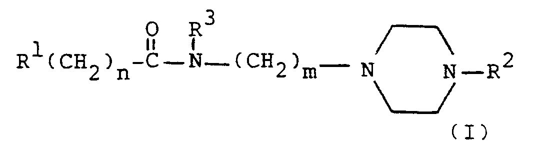
wherein R¹ is 1-adamantyl, 3-methyl-1-adamantyl, 3-noradamantyl, unsubstituted or substituted -2-indolyl, 3-indolyl, 2-benzofuranyl or 3-benzofuranyl wherein the substituents are selected from lower alkyl, lower alkoxy and halo; R² is unsubstituted or substituted phenyl, benzyl, pyridinyl, pyrimidinyl or pyrazinyl, wherein the substituents are selected from lower alkyl, lower alkoxy, trifluoromethyl and halo; R³ is H or lower alkyl of 1 to 3 carbon atoms; n is the integer 0 or 1; and m is an integer from 2 to 5 and the pharmaceutically acceptable salts thereof, in the manufacture of a medicament for the treatment of anxiety, depression or psychoses.

The invention also provides compounds having formula I as defined above with the proviso that when R¹ is substituted or unsubstituted 2-benzofuranyl and n is 0 then R² is other than phenyl or phenyl substituted by halogen, alkyl of 1 to 6 carbon atoms, alkoxy of 1 to 6 carbon atoms or trifluoromethyl

Examples of R² are phenyl, methoxyphenyl (eg. 2-methoxyphenyl), chlorophenyl (eg. 3-chlorophenyl), 3-(trifluoromethyl)phenyl, pyridinyl and pyrimidinyl (eg. 2-pyrimidinyl).

Examples of m are 2 and 3. Preferred among the compounds are those of the general structure (I) wherein R¹ is 1-adamantyl, 3-methyl-1-adamantyl, 2-indolyl, 2-benzofuranyl; R² is unsubstituted or substituted phenyl, wherein the substituents are selected from methoxy and chloro, pyridinyl or 2-pyrimidinyl; R³ is H; n is the integer 0 or 1; m is the integer 2 or 3 and the pharmaceutically acceptable salts thereof.

The most preferred compounds of the present invention are designated
N-[3-[4-(2-methoxyphenyl)-1-piperazinyl]propyl]tricyclo[3.3.1.1^{3,7}] decane-1-carboxamide;
N-[2-[4-(2-pyrimidyl)-1-piperazinyl]ethyl]tricyclo[3.3.1.1^{3,7}]decane-1-carboxamide;
N-[2-[4-(2-methoxyphenyl)-1-piperazinyl]ethyl]tricyclo[3.3.1.1^{3,7}] decane-1-carboxamide;
N-[2-[4-(3-chlorophenyl)-1-piperazinyl]ethyl]tricyclo[3.3.1.1^{3,7}]decane-1-carboxamide;
N-[2-[4-(2-pyrimidyl-1-piperazinyl]ethyl]benzofurane-2-carboxamide;
N-[3-[4-(2-methoxyphenyl)-1-piperazinyl]propyl]benzofurane-2-carboxamide;
N-[3-[4-(2-methoxyphenyl)-1-piperazinyl]propyl]-(1H)-indole-2-carboxamide;
N-[3-[4-(3-chlorophenyl)-1-piperazinyl]propyl]tricyclo[3.3.1.1^{3,7}] decane-1-carboxamide;
N-[2-[4-(2-pyrimidyl)-1-piperazinyl]ethyl]-3-methyltricyclo[3.3.1.1^{3,7}]decane-1-acetic acid carboxamide;
N-[3-(4-(2-pyrimidyl)-1-piperazinyl]propyl]tricyclo[3.3.1.1^{3,7}]decane-1-carboxamide;
N-[2-[4-(3-chlorophenyl)-1-piperazinyl]ethyl]-3-methyltricyclo[3.3.1.1^{3,7}]decane-1-acetic acid carboxamide;
N-[2-[4-(2-methoxyphenyl)-1-piperazinyl]ethyl]-3-methyltricyclo[3.3.1.1^{3,7}]decane-1-acetic acid carboxamide;
N-[2-[4-(2-pyrimidyl)-1-piperazinyl]ethyl]hexahydro-2,5-methanopentalene-3a(1H)-carboxamide;
N-[2-[4-(2-methoxyphenyl)-1-piperazinyl]ethyl]hexahydro-2,5-methanopentalene-3a(1H)-carboxamide;
N-[2-[4-(3-chlorophenyl)-1-piperazinyl]ethyl]hexahydro-2,5-methanopentalene-3a(1H)-carboxamide;
N-[2-[4-[3-(trifluoromethyl)phenyl]-1-piperezinyl]ethyl]hexahydro-2,5-methanopentalene-3a(1H)-carboxamide;
N-[3-[4-(2-pyrimidyl)-1-piperazinyl]propyl]hexahydro-2,5-methanopentalene-3a(1H)-carboxamide;
N-[3-[4-(2-methoxyphenyl)-1-piperazinyl]propyl]hexahydro-2,5-methanopentalene-3a(1H)-carboxamide;
N-[3-[4-(3-chlorophenyl)-1-piperazinyl]propyl]hexahydro-2,5-methanopentalene-3a(1H)-carboxamide;
and the pharmaceutically acceptable salts thereof.

The term "lower alkyl" refers to moieties having 1 to 6 carbon atoms in the carbon chain. The term "alkoxy" refers to moieties having 1 to 6 carbon atoms. The term "halo" refers to fluoro, chloro and bromo.

The compounds of the invention can form pharmacologically acceptable salts from pharmacologically acceptable organic and inorganic acids such as hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic and benzenesulfonic.

The compounds of this invention which demonstrated selectivity at the 5-HT_{1A} and 5-HT₂ versus D₂ receptor binding sites are useful as potential anxiolytic-antidepressant agents.

In addition, compounds of this invention with equal high affinity for the 5-HT_{1A} and D₂-receptor binding sites are useful as mixed antipsychotic-anxiolytic agents.

Compounds of this invention which demonstrated central cholinergic activity are useful in the treatment of senile dementia of the Alzheimer type (SDAT) and Huntingdon's chorea.

Compounds of this invention can be prepared by a variety of synthetic routes by building the molecule up from smaller constituent molecules.

Accordingly this invention provides a process for preparing a compound of formula (I) as defined above which comprises one of the following:
(a) acylating a compound of formula 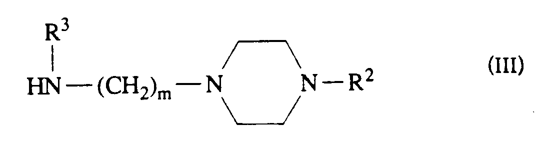 with an acylating agent containing the group R⁵ wherein R⁵ is:

   R¹-(CH₂)ₙ-C(O)- (IIa)

   wherein n, m, R¹, R², and R³ are as defined above to give a compound of formula (I);
   or
(b) a cyclic amine having the formula (IV) 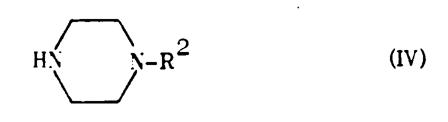 wherein R² is as defined above or a salt thereof is alkylated to introduce the substituted alkyl group having the formula (V) 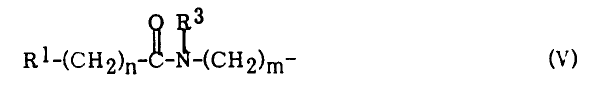 by reaction with a compound having the formula R⁴-Y (wherein R⁴ is the group having formula (V) and Y is a leaving group, for example halo such as chloro or bromo or aryl- or alkyl-sulphonyloxy);
   or
(c) a cyclic amine having the formula (IV) as defined above or a salt thereof is subjected to reductive alkylation with an aldehyde having the formula (VI) 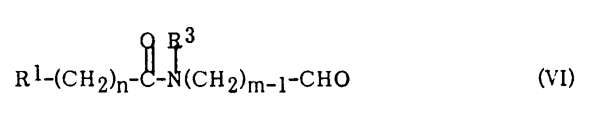 wherein n, R¹, R³ and m are as defined above;
   or
(d) a compound having formula (I) is converted into an acid addition salt thereof by addition of an acid or an acid addition salt of a compound having formula (I) is subjected to neutralisation to form the compound having formula (I).

With reference to process step (a) above, acylation is conveniently carried out under basic conditions using methods generally known for preparing secondary or tertiary amides. Examples of acylating agents are reactive derivatives of acids of formula R⁵OH such as acid halides, eg. the chloride, azide, anhydride, mixed anhydride (eg. forced with carbonyldiimidazole) or activated esters (eg. 1-benzotriazolyl, 2,3,4-trichlorophenyl or p-nitrophenyl) or O-acyl ureas obtained from carbodiimides such as dialkylcarbodiimides, eg. dicyclohexylcarbodiimide. Descriptions of methods for forming amides are given in the literature - see for example "The Chemistry of Amides" Interscience Publisher, 1970, Chapter beginning at p 73 from the series "The Chemistry of Functional Groups" edited by Saul Patai and books on peptide chemistry - eg. The Practice of Peptide Synthesis, by M. Bodanszky and A. Bodanszky, Springer Verlag, 1984. Volume 21 of the series Reactivity and Structure Concepts in Organic Chemistry.

Process step (b) may be carried out in the conventional manner for the preparation of tertiary amines by alkylation of secondary amines. In particular the reaction may be carried out in a suitable solvent, eg. dimethylformamide in the presence of an inorganic base or a tertiary amine, eg. triethylamine.

Process step (c) may be carried out in the conventional manner for the preparation of tertiary amines from secondary amines and aldehydes by reductive alkylation. The reductive alkylation may be carried out with hydrogen and platinum catalyst or using sodium cyanoborohydride.

Starting materials for the processes described above are in general known compounds or can be prepared by methods known for analogous compounds where necessary by building up the molecule from readily available starting materials.

Piperazines of formula (IV) can be prepared by known methods, eg. reaction of bis-(2-chloroethyl)amine with an amine or aniline of formula H₂NR².

Compounds of formula R⁴-Y wherein R⁴ is 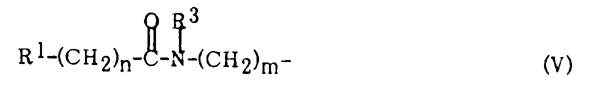
can be prepared by (a) acylating an hydroxy amine of formula

HR³N-(CH₂)ₘOH

with an acylating agent containing the group 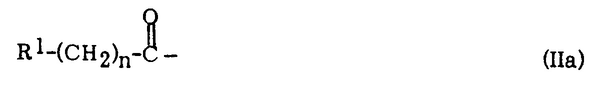
to give a compound of formula R⁴OH and (b) converting the terminal OH group to a leaving group by known methods, eg. halogenating (using SOCl₂) or sulphonating. Compounds of formula (VI) may be prepared by oxidising compounds of formula R⁴OH, eg. using pyridinium chlorochromate in dichloromethane.

In a preferred process 1-adamantane carboxylic acid halide, noradamantane carboxylic acid halide, indolecarboxylic acid halide or benzofurancarboxylic acid halide of formula (II) may be conveniently reacted with the appropriately substituted aminoalkyl piperazine of formula (III) 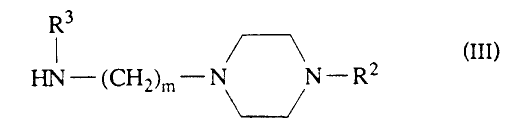
in CH₂Cl₂ and the presence of a suitable base, such as triethylamine, to obtain the desired final product (I). 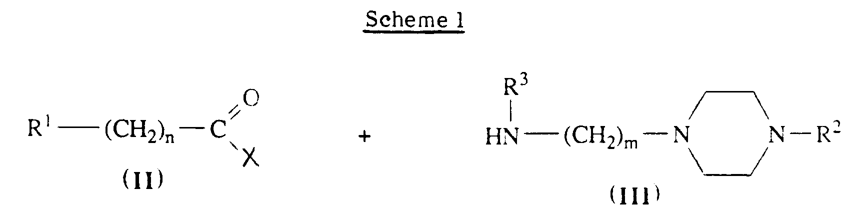
wherein X is a halide and R¹, R², and R³, and n are as defined above. For particular case when R² is benzyl, hydrogenation of (I) followed by treatment of the product R² is H with 2-chloropyrimidine permits an alternative synthesis of (I) where R² is 2-pyrimidinyl.

Of course, other methods of preparation, which will occur to those skilled in the art, may also be employed to prepare the compounds of the invention.

The starting material used in the above-described preparative routes are commercially available, or can be made according to procedures taught in the chemical literature.

The compounds of the invention may exist either in the form of the free base or the pharmacologically acceptable salts. Methods for converting one such form to another will be obvious to one skilled in the chemical arts.

The compounds of the invention display a preclinical pharmacological profile like that of the compound gepirone (4,4-dimethyl-1-[4-[4-(2-pyrimidinyl)-1-piperazinyl]-butyl]-2,6-piperidinedione) and ritanserin 6-[2-[4-[bis(4-fluorophenyl)methylene]-1-piperidinyl]-7-methyl-5H-thiazolo[3,2-a]pyrimidin-5-one.

Gepirone and ritanserin have demonstrated clinical activity in anxiolytic and antidepressant paradigms and have also displayed a unique clinical anxio-selective profile, whereby their efficacy in the treatment of anxiety neuroses is comparable to the benzodiazepine diazepam. Additionally, most chronically used antipsychotic drugs cause extra-pyramidal side effects, such as pseudo-parkinsonism, tardive dyskinesia and the like. Ideally, treatment of depression, psychoses and anxiety should be free of any undesirable side effects. The compounds of the invention, in a manner similar to ritanserin and buspirone, may display preclinical anxiolytic and antidepressant activities with expected minimal side effects. Based on their buspirone-like profile, the compounds of the invention can be considered of clinical value in treating anxiety neuroses. Moreover, based on the central cholinergic activity, the compounds of the present invention are useful in the treatment of central cholinergic dysfunction attending senile dementia of the Alzheimer type (SDAT).

When employed as anxiolytic/antidepressant, the effective dosage of the active substances for such treatment will vary according to the particular compound being employed, and the severity and nature of the condition being treated. Therapy should be initiated at lower doses, the dosage thereafter being increased, if necessary, to produce the desired effect. In general, the compounds of the invention are most desirably administered at a concentration that will generally afford effective results without causing any harmful or deleterious side effects.

When the compounds of the invention are employed as anxiolytic/antidepressant or anxiolytic/antipsychotic agents, they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methylcellulose, sodium carboxymethylcellulose, low melting wax, cocoa butter and the like. Diluents, flavoring agents, solubilizers, lubricants, suspending agents, binders, tablet-disintegrating agents and the like may be employed. The compounds may be encapsulated with or without other carriers. In all cases, the proportion of active ingredients in said compositions both solid and liquid will be sufficient at least to impart the desired activity thereto on oral administration. The compounds may also be injected parenterally in which case they are used in the form of a sterile solution containing other solutes, for example, enough saline or glucose to make the solution isotonic. Accordingly this invention provides a pharmaceutical composition comprising a compound of formula (I) or a pharmaceutically acceptable salt thereof and a pharmaceutically acceptable carrier. The antidepressant activity of the compounds of the invention and their expected lack of extrapyramidal side effects may be demonstrated by standard pharmacological procedures, which are described more fully in the examples given hereafter.

The following examples show the preparation and pharmacological testing of compounds within the invention.

### EXAMPLE 1

### N-[3-[4-(2-Methoxyphenyl)-1-piperazinyl]propyl]tricyclo[3.3.1.1^{3,7}]decan-1-carboxamide

To a stirred solution of [4-(2-methoxyphenyl)piperazino]propylamine (2.5 g, 0.01 mol) in 50 mL of methylene chloride, adamantane-1-carboxylic acid chloride (2.02 g, 0.010 mol) and triethylamine (2 g, 0.02 mol) were added. Stirring was continued at room temperature overnight. The methylene chloride solution was washed with water, dried over anhydrous sodium sulfate and evaporated under reduced pressure. The remaining residue was subjected to preparative HPLC. In repeated preparations, the residue was dissolved in ethyl acetate (10 mL) and subjected to flash chromatography using a 9 inch column of silica gel and ethyl acetate as the eluent. The title compound (TLC R_{f} = 0.53 in 30% methanol/ethyl acetate system) was separated and converted to the hydrochloride hemihydrate salt with ethanolic HCl (2.3 g), m.p. 186-190°C.

| | | | |
|---|---|---|---|
| Anal. Calcd. C₂₅H₃₇N₃O₂·HCl·0.5H₂O: | C, 65.69; | H, 8.60; | N, 9.19% |
| Found: | C, 66.04; | H, 8.26; | N, 9.18%. |

### EXAMPLE 2

### N-[2-[4-(2-Pyrimidyl)-1-piperazinyl]ethyl]tricyclo[3.3.1.1^{3,7}]decane-1-carboxamide

To a stirred solution of [4-(2-pyrimidinyl)piperazino]ethylamine (2.0 g, 0.01 mol) in 50 mL of methylene chloride, adamantane-1-carboxylic acid chloride (3.6 g, 0.018 mol) and triethylamine (2.9 g, 0.015 mol) were added. Stirring was continued at room temperature overnight. The methylene chloride solution was washed with water, dried over anhydrous sodium sulfate and evaporated under reduced pressure. The remaining residue was dissolved in ethyl acetate (10 mL) and subjected to column chromatography using silica gel and ethyl acetate as the eluent. The title compound was separated and converted to the dihydrochloride hydrate salt with ethanolic HCl (2.4 g), m.p. 237-239°C.

| | | | |
|---|---|---|---|
| Anal. Calcd. C₂₁H₃₁N₅O·2HCl·H₂O: | C, 54.80; | H, 7.66; | N, 15.21% |
| Found: | C, 54.99; | H, 6.91; | N, 15.14%. |

### EXAMPLE 3

### N-[2-[4-(2-Methoxyphenyl)-1-piperazinyl]ethyl]tricyclo[3.3.1.1^{3,7}]decane-1-carboxamide

To a stirred solution of [4-(2-methoxyphenyl)piperazino]ethylamine (2.53 g, 0.01 mol) in 50 mL of methylene chloride, adamantane-1-carboxylic acid chloride (3 g, 0.02 mol) and triethylamine (2 g, 0.02 mol) were added. Stirring was continued at room temperature overnight. The methylene chloride solution was washed with water, dried over anhydrous sodium sulfate and evaporated under reduced pressure. The remaining residue was subjected to preparative HPLC. In repeated preparations, the residue was dissolved in ethyl acetate (10 mL) and subjected to flash chromatography using a 9 inch column of silica gel and ethyl acetate as the eluent. The title compound (TLC R_{f} = 0.65 in 30% methanol/ethyl acetate system) was separated and converted to the dihydrochloride sesquihydrate salt with ethanolic HCl (1.6 g), m.p. 206-212°C.

| | | | |
|---|---|---|---|
| Anal. Calcd. C₂₄H₃₅N₃O₂·2HCl·0.75H₂O: | C, 59.56; | H, 8.02; | N, 8.68% |
| Found: | C, 59.65; | H, 7.38; | N, 8.65%. |

### EXAMPLE 4

### N-[2-[4-(3-Chlorophenyl)-1-piperazinyl]ethyl]tricyclo[3.3.1.1^{3,7}]decane-1-carboxamide

To a stirred solution of [4-(m-chlorophenyl)piperazino]ethylamine (2.5 g, 0.01 mol) in 50 mL of methylene chloride, adamantane-1-carboxylic acid chloride (5 g, 0.018 mol) and triethylamine (3.6 g, 0.018 mol) were added. Stirring was continued at room temperature overnight. The methylene chloride solution was washed with water, dried over anhydrous sodium sulfate and evaporated under reduced pressure. The remaining residue was dissolved in ethyl acetate (10 mL) and subjected to column chromatography using silica gel and ethyl acetate as the eluent. The title compound was separated and converted to the dihydrochloride salt with ethanolic HCl (0.7 g), m.p. 210-213°C.

| | | | |
|---|---|---|---|
| Anal. Calcd. C₂₃H₃₂ClN₃O·2HCl: | C, 58.17; | H, 7.22; | N, 8.45% |
| Found: | C, 57.70; | H, 7.02; | N, 8.61%. |

### EXAMPLE 5

### N-[2-[4-(2-Pyrimidyl)-1-piperazinyl]ethyl]benzofurane-2-carboxamide

To a stirred solution of [4-(2-pyrimidinyl)piperazino]ethylamine (2.0 g, 0.01 mol) in 50 mL of methylene chloride, benzofurane-2-carboxylic acid chloride (2.6 g, 0.014 mol) and triethylamine (2 g, 0.02 mol) were added. Stirring was continued at room temperature overnight. The methylene chloride solution was washed with water, dried over anhydrous sodium sulfate and evaporated under reduced pressure. The remaining residue was subjected to preparative HPLC. The title compound was separated and converted to the dihydrochloride hydrate salt with ethanolic HCl (0.5 g), m.p. 193°C.

| | | | |
|---|---|---|---|
| Anal. Calcd. C₁₉H₂₁N₅O₂·2HCl·H₂O: | C, 51.59; | H, 5.70; | N, 15.83% |
| Found: | C, 51.42; | H, 5.50; | N, 15.71%. |

### EXAMPLE 6

### N-[3-[4-(2-Methoxyphenyl)-1-piperazinyl]propyl]benzofurane-2 carboxamide

To a stirred solution of [4-(2-methoxyphenyl)piperazino]propylamine (1.0 g, 0.04 mol) in 50 mL of methylene dichloride, benzofurane-2-carboxylic acid chloride (1.01 g, 0.0056 mol) and triethylamine (1 g, 0.01 mol) were added. Stirring was continued at room temperature overnight. The methylene chloride solution was washed with water, dried over anhydrous sodium sulfate and evaporated under reduced pressure. The remaining residue was subjected to preparative HPLC using silica gel column and ethyl acetate as the eluent. The title compound was separated and converted to the dihydrochloride salt with ethanolic HCl (0.9 g), m.p. 228-232°C.

| | | | |
|---|---|---|---|
| Anal. Calcd. C₂₃H₂₇N₃O₃·2HCl: | C, 59.23; | H, 6.27; | N, 9.01% |
| Found: | C, 59.59; | N, 6.12; | N, 8.84%. |

### EXAMPLE 7

### N-[3-[4-(2-Methoxyphenyl)-1-piperazinyl]propyl]-(1H)-indole-2 carboxamide

The title compound was prepared by stirring [4-(2-methoxyphenyl)piperazino]propylamine (1.4 g, 0.005 mol), indole-2-carboxylic acid chloride (1.0 g, 0.005 mol) and triethylamine (1 g, 0.01 mol) in 50 mL of CH₂Cl₂ for 24 hours. The methylene chloride solution was washed with water, dried (anhydrous Na₂SO₄) and evaporated under reduced pressure. The remaining residue was subjected to preparative HPLC over silica gel column and using ethyl acetate as the eluent and the desired product (TLC R_{f} = 0.65 in 30% methanol/ethyl acetate system) was separated and converted to the dihydrochloride dihydrate salt with ethanolic HCl, m.p. 173-178°C.

| | | | |
|---|---|---|---|
| Anal. Calcd. C₂₃H₂₈N₄O₂·2HCl·2H₂O: | C, 55.49; | H, 6.41; | N, 10.73% |
| Found: | C, 55.09; | H, 6.83; | N, 11.17%. |

### EXAMPLE 8

### N-[3-[4-(3-Chlorophenyl)-1-piperazinyl]propyl]tricyclo[3.3.1.1^{3,7}]decane-1-carboxamide

To a stirred solution of [4-(3-chlorophenyl)piperazino]propylamine (1.28 g, 0.005 mol) in 50 mL of methylene chloride, adamantane-1-carboxylic acid chloride (1 g, 0.005 mol) and triethylamine (1 g, 0.01 mol) were added. Stirring was continued at room temperature overnight. The methylene chloride solution was washed with water, dried over anhydrous sodium sulfate and evaporated under reduced pressure. The remaining residue was subjected to preparative HPLC using silica gel column and ethyl acetate as the eluent. The title compound was separated and converted to the dihydrochloride salt with ethanolic HCl (1.5 g), m.p. 209-210°C.

| | | | |
|---|---|---|---|
| Anal. Calcd. C₂₄H₃₄ClN₃O·2HCl·1.50H₂O: | C, 55.87; | H, 4.72; | N, 8.14% |
| Found: | C, 56.05; | H, 7.62; | N, 7.69%. |

### EXAMPLE 9

### N-[2-[4-(2-Pyrimidyl)-1-piperazinyl]ethyl]-3-methyltricyclo[3.3.1.1^{3,7}]decane-1-acetic Acid Carboxamide

The title compound was prepared by stirring [4-(2-pyrimidinyl]-piperazino]ethylamine (2.0 g, 0.009 mol), 3-methyladamantane-1-acetic acid bromoethyl ester (2.34 g, 0.01 mol) and triethylamine (1.0 g, 0.015 mol) in 50 mL of CH₂Cl₂ for 24 hours. The methylene chloride solution was washed with water, dried (anhydrous Na₂SO₄) and removed under reduced pressure. The remaining residue was subjected to preparative HPLC over silica gel column using ethyl acetate as the eluent and the desired product was separated and converted to the dihydrochloride, hemihydrate salt with ethanolic HCl, m.p. 248-251°C.

| | | | |
|---|---|---|---|
| Anal. Calcd. C₂₃H₃₅N₅O·2HCl·0.50H₂O: | C, 57.61; | H, 7.99; | N, 14.61% |
| Found: | C, 57.08; | H, 7.70; | N, 14.39%. |

### EXAMPLE 10

### N-[3-[4-(2-Pyrimidyl)-1-piperazinyl]propyl]tricyclo[3.3.1.1^{3,7}]decane-1-carboxamide

To a stirred solution of [4-(2-pyrimidinyl)piperazino]propylamine (1.12 g, 0.005 mol) in 50 mL of methylene chloride, adamantane-1-carboxylic acid chloride (1 g, 0.005 mol) and triethylamine (1.0 g, 0.01 mol) were added. Stirring was continued at room temperature overnight. The methylene chloride solution was washed with water, dried over anhydrous sodium sulfate and evaporated under reduced pressure. The remaining residue was subjected to preparative HPLC using a column of silica gel and ethyl acetate as the eluent. The title compound was separated and converted to the hydrochloride, hydrate salt with ethanolic HCl (1.6 g), m.p. 146-148°C.

| | | | |
|---|---|---|---|
| Anal. Calcd. C₂₂H₃₃N₅O·HCl·H₂O: | C, 60.32; | H, 8.29; | N, 15.99% |
| Found: | C, 60.57; | H, 8.49; | N, 15.10%. |

### EXAMPLE 11

### N-[2-[4-(3-Chlorophenyl)-1-piperazinyl]ethyl]-3-methyltricyclo[3.3.1.1^{3,7}]decane-1-acetic Acid Carboxamide

The title compound was prepared by stirring [4-(3-chlorophenyl)piperazinyl]ethylamine (2.5 g, 0.010 mol), 3-methyladamantane-1-acetic acid bromoethyl ester (2.67 g, 0.01 mol) and triethylamine (2 g, 0.025 mol) in 50 mL of CH₂Cl₂ for 24 hours. The methylene chloride solution was washed with water, dried (anhydrous Na₂SO₄) and evaporated under reduced pressure. The remaining residue was subjected to preparative HPLC over silica gel using ethyl acetate as the eluent and the desired product was separated and converted to the dihydrochloride, 2 1/2 hydrate salt with ethanolic HCl, m.p. 178-182°C.

| | | | |
|---|---|---|---|
| Anal. Calcd. C₂₅H₃₆ClN₃O·2HCl·2.50H₂O: | C, 54.80; | H, 7.91; | N, 7.67% |
| Found: | C, 54.44; | H, 6.97; | N, 7.49%. |

### EXAMPLE 12

### N-[2-[4-(2-Methoxyphenyl)-1-piperazinyl]ethyl]-3-methyltricyclo[3.3.1.1^{3,7}]decane-1-acetic Acid Carboxamide

To a stirred solution of [4-(2-methoxyphenyl)piperazino]ethylamine (2.0 g, 0.008 mol) in 50 mL of methylene chloride, 3-methyladamantane-1-carboxylic acid chloride (2.1 g, 0.004 mol) and triethylamine (1 g, 0.01 mol) were added. Stirring was continued at room temperature overnight. The methylene chloride solution was washed with water, dried over anhydrous sodium sulfate and evaporated under reduced pressure. The remaining residue was subjected to preparative HPLC using a column of silica gel and ethyl acetate as the eluent. The title compound was separated and converted to the dihydrochloride salt with ethanolic HCl (1.3 g), m.p. 194-197°C.

| | | | |
|---|---|---|---|
| Anal. Calcd. C₂₆H₃₉N₃O₂·2HCl: | C, 62.64; | H, 8.29; | N, 8.43% |
| Found: | C, 62.07; | H, 7.75; | N, 8.76%. |

### EXAMPLE 13

### N-[2-[4-(2-Pyrimidyl)-1-piperazinyl]ethyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 2-[1-(2-pyrimidyl)-4-piperazinyl]aminoethane (0.75 g, 3.6 x 10⁻³ mol) in 25 mL of chloroform was then added, and the resulting reaction mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 30% methanol in ethyl acetate solvent system, Rf = 0.45) was isolated by gravity chromatography on silica gel and converted to the dihydrochloride hemihydrate salt (0.84 g, 50% yield), m.p. 210-211°C.

| | | | |
|---|---|---|---|
| Anal. Calcd. C₂₀H₂₉N₅O·2HCl·0.50H₂O: | C, 54.87; | H, 7.36; | N, 16.00% |
| Found: | C, 54.69; | H, 7.13; | N, 16.56%. |

### Example 14

### N-[2-[4-(2-Methoxyphenyl)-1-piperazinyl]ethyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 2-[1-(2-methoxyphenyl)-4-piperazinyl]aminoethane (0.85 g, 3.6 x 10⁻³ mol) in 25 mL of chloroform was then added, and the resulting reaction mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 20% methanol in ethyl acetate solvent system, Rf = 0.47) was isolated by gravity chromatography on silica gel and converted to the dihydrochloride salt (0.84 g, 56% yield), m.p. 192-193°C.

| | | | |
|---|---|---|---|
| Anal. Calcd. C₂₃H₃₃N₃O₂·2HCl: | C, 60.46; | H, 7.66; | N, 9.20% |
| Found: | C, 60.06; | H, 7.48; | N, 9.14%. |

### Example 15

### N-[2-[4-(3-Chlorophenyl)-1-piperazinyl]ethyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 2-[1-(3-chlorophenyl)-4-piperazinyl]aminoethane (0.86 g, 3.6 x 10⁻³ mol) in 25 mL of chloroform was then added, and the resulting mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 20% methanol in ethyl acetate solvent system, Rf = 0.55) was isolated by gravity chromatography on silica gel and converted to the dihydrochloride salt (0.75 g, 43% yield), m.p. 226-227°C.

| | | | |
|---|---|---|---|
| Anal. Calcd. C₂₂H₃₀ClN₃O·2HCl: | C, 57.33; | H, 6.99; | N, 9.12% |
| Found: | C, 57.58; | H, 7.10; | N, 9.12%. |

### Example 16

### N-[2-[4-[3-Trifluoromethyl)phenyl]-1-piperazinyl]ethyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 2-[1-(3-chlorophenyl)-4-piperazinyl]aminoethane (0.98 g, 3.6 x 10⁻³ mol) in 25 mL of chloroform was then added, and the resulting reaction mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 10% methanol in ethyl acetate solvent system, Rf = 0.40) was isolated by preparative high pressure liquid chromatography (HPLC) on silica gel using a 0% to 5% gradient of methanol in ethyl acetate, and converted to the dihydrochloride salt (0.88 g, 50% yield), m.p. 222-223°C.

| | | | |
|---|---|---|---|
| Anal. Calcd. C₂₃H₃₀F₃N₃O·2HCl: | C, 55.87; | H, 6.52; | N, 8.50% |
| Found: | C, 56.12; | H, 6.90; | N, 8.42%. |

### Example 17

### N-[3-[4-(2-Pyrimidyl)-1-piperazinyl]propyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 3-[1-(2-pyrimidyl)-4-piperazinyl]aminopropane (0.80 g, 3.6 x 10⁻³ mol) in 25 mL of chloroform was then added, and the resulting reaction mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 30% methanol in ethyl acetate solvent system, Rf = 0.44) was isolated by preparative HPLC on silica gel using a 2% to 5% gradient of methanol in ethyl acetate, and converted to the dihydrochloride salt (0.65 g, 42% yield), m.p. 229-230°C.

| | | | |
|---|---|---|---|
| Anal. Calcd. C₂₁H₃₁N₅O·2HCl: | C, 57.01; | H, 7.52; | N, 15.83% |
| Found: | C, 56.64; | H, 7.51; | N, 15.49%. |

### Example 18

### N-[3-[4-(2-Methoxyphenyl)-1-piperazinyl]propyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 3-[1-(2-methoxyphenyl)-4-piperazinyl]aminopropane (0.91 g, 3.6 x 10⁻³ mol) in 25 mL of chloroform was then added, and the resulting reaction mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 30% methanol in ethyl acetate solvent system, Rf = 0.45) was isolated by gravity chromatography on silica gel and converted to the dihydrochloride salt (0.96 g, 56% yield), m.p. 201-202°C.

| | | | |
|---|---|---|---|
| Anal. Calcd. C₂₄H₃₅N₃O₂·2HCl: | C, 61.27; | H, 7.93; | N, 8.93% |
| Found: | C, 60.88; | H. 7.81; | N, 8.81%. |

### Example 19

### N-[3-[4-(3-Chlorophenyl)-1-piperazinyl]propyl]hexahydro-2,5-methanopentalene-3a(1H)-carboxamide

To a stirred solution of noradamantane-3-carboxylic acid (0.6 g, 3.6 x 10⁻³ mol) in 25 mL of chloroform under a dry nitrogen atmosphere was added carbonyldiimidazole (0.58 g, 3.6 x 10⁻³ mol). The resulting solution was stirred at ambient temperature for three hours, during which time a gas (CO₂) was evolved. A solution of 3-[1-(3-chlorophenyl)-4-piperazinyl]aminopropane (0.92 g, 3.6 x 10⁻³ mol) in 25 mL of chloroform was then added, and the resulting reaction mixture was stirred under nitrogen at ambient temperature for 2 days. The mixture was diluted to 150 mL with chloroform, washed with three-one hundred mL portions of water, dried over anhydrous sodium sulfate, and concentrated on a rotary evaporator. The desired product (TLC on silica using a 20% methanol in ethyl acetate solvent system, Rf = 0.52) was isolated by gravity chromatography on silica gel and converted to the hydrochloride salt (0.74 g, 47% yield), m.p. 236-237°C.

| | | | |
|---|---|---|---|
| Anal. Calcd. C₂₃H₃₂ClN₃O·HCl: | C, 63.00; | H, 7.59; | N, 9.58% |
| Found: | C, 62.84; | H, 7.66; | N, 9.57%. |

### Example 20

The compounds of the invention were evaluated for their ability to inhibit limbic D-2 dopamine receptor binding. This in vitro assay measures the ability of the compounds tested to bind to the dopamine receptor sites. Those compounds which exhibit a high binding effect have a high potential to display antipsychotic effects.

The test is carried out as follows:
Several rats are decapitated and the brains are rapidly removed. Limbic brain tissue (nucleus accumbens, septal area, olfactory tubercle) is dissected and homogenized on ice in 9 volumes of buffer (50 mM Tris-HCl, 120 mM NaCl, 5mM KCl, 1 mM CaCl₂, 1 mM MgCl₂, 0.1% L-ascorbic acid, 10 µM pargyline HCl, pH 7.1) using a Polytron homogenizer at setting 5 for three 15-second bursts. The homogenate is then diluted 4-fold with buffer and centrifuged at 30,000 x g for 20 minutes, and the supernatant is discarded. The pellet is resuspended in the same volume of buffer and recentrifuged as before, again discarding the supernatant. This pellet is then resuspended in the same volume of buffer used in the homogenization, and the protein content of this preparation is assayed by the Lowry method. The homogenate is stored frozen at -70°C until use.

Thirty µL of the homogenate (0.2-0.3 mg protein/sample) are incubated with 0.3 nM ³H-spiroperidol (New England Nuclear) and various concentrations of test drug in a final volume of 1 mL of the above buffer for 10 minutes in a 37°C water bath. All tubes contained 30 nM ketanserin to exclude binding to 5-HT₂ receptors. At the end of the incubation, 3 mL of cold 50 mM Tris-HCl, pH 7.7, are added to each tube, and the contents are rapidly vacuum-filtered through Whatman GF/B glass-fiber filters. The filters are then rapidly washed 3 times with 3 mL of the same buffer, placed in scintillation vials, and shaken for 15 minutes with 10 mL of Hydrofluor (National Diagnostics) scintillation cocktail. The vials are then counted in a Packard 460CD scintillation counter.

Specific binding is defined as total binding less binding in the presence of 10 µM sulpiride. Binding in the presence of various concentrations of test drug is expressed as a percent of specific binding when no drug is present. These results are then plotted as logit % binding vs. log concentration of test drug. Linear regression analysis then yields a straight line with 95% confidence limits from which an IC₅₀ can be inversely predicted. Kᵢ (inhibition constant) for the test drug is then calculated by the formula: 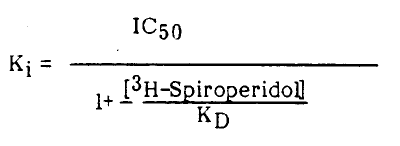
where K_{D}=0.3 nM for spiroperidol binding

| Standard Compounds: | Kᵢ and 95% confidence interval |
|---|---|
| Haloperidol | 4.0 (3.0 - 5.6) nM |
| Clozapine | 34 (23 - 54) nM |
| Fluphenazine | 4.5 (3.6 - 5.6) nM |
| Sulpiride | 376 (174 - 5000) nM |

The results of testing of some of the compounds of the invention, and the prior art compound gepirone (4,4-dimethyl-1-[4-[4-(2-pyrimidinyl)-1-piperaziny]-butyl]-2,6-piperidinedione) in this assay are presented in Table 1.

The results show that compounds of the invention display affinity for the D₂ receptor, evidencing potential for antipsychotic effects.

### Example 21

The in vitro inhibition of 5-HT_{1A} serotonin receptor binding is used to determine whether the test compounds possess affinity at 5-HT_{1A} receptors and whether there is an indication of gepirone like anxiolytic activity.

The assay is carried out as follows:
Hippocampal tissue from male Sprague Dawley rats is dissected and homogenized on ice in 40 volumes of buffer A (50 mM Tris HCl, pH = 7.7) using a Polytron homogenizer at setting 5 for 3 x 15 second bursts. The homogenate is then centrifuged at 20,000 rpm (RC5-B; 50,000 g) and the supernatant discarded. The pellet is resuspended in 40 volumes of the same buffer and incubated at 37°C for 10 minutes to aid in the removal of endogenous serotonin. The homogenate is then centrifuged (as above) and the supernatant discarded. The pellet is then resuspended in 100 volumes of buffer B (50 mM Tris HCl, pH = 7.7 containing 0.1% ascorbate, 10 M pargyline and 4 mM CaCl₂) and sonicated. An aliquot is taken for protein determination by the Lowry method and the remainder stored frozen at -70°C until used.

The homogenate (50 µL; 0.4-0.6 mg protein/sample) is incubated with 100 µL (1.5-1.8 nM) ³H-8-hydroxy-2-(di-n-propylamino)tetraline (³H-8-OH-DPAT) in a final volume of 2 mL of buffer for 10 minutes at 37°C. At the end of the incubation, 3 mL of cold buffer A are added to each tube, and the contents rapidly filtered through Whatman GF/B glass filters. The filters are then rapidly washed 2 times with 3 mL of the same buffer, placed in scintillation vials, and shaken for 15 minutes with 10 mL of Hydrofluor (National Diagnostics) scintillation cocktail. The vials are then counted in a Packard 460 CD scintillation counter.

Specific binding is defined as total binding less binding in the presence of excess unlabeled serotonin (1 µM). Binding in the presence of various concentrations of test drug is expressed as a percent of specific binding when no drug is present. These results are then plotted as logit % binding vs. log concentration of test drug. Linear regression analysis then yields a straight line with 95% confidence limits from which an IC₅₀ can be inversely predicted. Kᵢ (inhibition constant) for the test drug is then calculated by the formula: 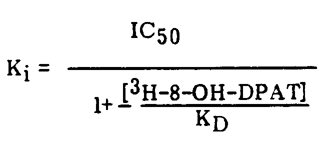
where K_{D}=1.8 nM for 8-OH-DPAT binding in hippocampus
When tested in this assay, the compounds of the invention gave the results set forth in Table 1.

The results show that compounds of the invention have a moderate to very high affinity for the 5-HT_{1A} receptor site, evidencing a high potential for anxiolytic activity.

### EXAMPLE 22

5-HT₂ inhibition of ³H-spiroperidol is determined in an analogous manner, employing rat brain cortex homogenate as the receptor tissue, following a modification of Fields et al, Brain Res., 136, 578 (1977); Yamamura et al, eds., Neurotransmitter Receptor Binding, Raven Press, N.Y. 1978; and Creese et al, Eur. J. Pharmacol., 49, 20 (1978).

The assay is carried out as follows:
Several rats are decapitated and the brains are rapidly removed. Cortical tissue is dissected and homogenized on ice in 9 volumes of buffer (50 mM Tris-HCl, 120 mM NaCl, 5 mM KCl, 1 mM CaCl₂, 1 mM MgCl₂, 0.1% L-ascorbic acid, 10 µM pargyline HCl, pH 7.1) using a Polytron homogenizer at setting 5 for 3 15-second bursts. The homogenate is then diluted 4-fold with buffer and centrifuged at 30,000 x g for 20 minutes and the supernatant is discarded. The pellet is resuspended in the same volume of buffer and recentrifuged as before, again discarding the supernatant. This pellet is then resuspended in the same volume of buffer used in the homogenization, and the protein content of this preparation is assayed by the Lowry method. The homogenate is stored frozen at -70°C until use.

Thirty µL of the homogenate (0.2-0.3 mg protein/sample) are incubated with 0.8 nM ³H-spiroperidol (New England Nuclear) and various concentrations of test drug in a final volume of 1 mL of the above buffer for 10 minutes in a 37°C water bath. At the end of the incubation, 3 mL of cold 50 mM Tris-HCl, pH 7.7, are added to each tube, and the contents are rapidly vacuum-filtered through Whatman GF/B glass-fiber filters. The filters are then rapidly washed 3 times with 3 mL of the same buffer, placed in scintillation vials, and shaken for 15 minutes with 10 mL of Hydrofluor (National Diagnostics) scintillation cocktail. The vials are then counted in a Packard 460CD scintillation counter.

Specific binding is defined as total binding less binding in the presence of 1 µM (+)butaclamol. Binding in the presence of various concentrations of test drug is expressed as a percent of specific binding when no drug is present. These results are then plotted as logit % binding vs. log concentration of test drug. Linear regression analysis then yields a straight line with 95% confidence limits from which an IC₅₀ can be inversely predicted. Kᵢ (inhibition constant) for the test drug is then calculated by the formula: 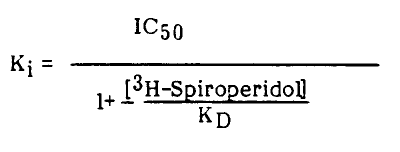
where K_{D}=0.8 nM for spiroperidol binding in cortex
When tested in this assay, the compounds of this invention gave the results set forth in Table 1.

### EXAMPLE 23

The M1 muscarinic receptor binding properties of the compounds of this invention were established as follows:

Homogenized rat hippocampus tissue is suspended in 0.32 M aqueous sucrose solution and centrifuged (747 x g for 10 minutes at 4°C) and the supernatent liquid is decanted and recentrifuged (18,677 x g for 20 minutes at 4°C). The resulting pellet is resuspended in the original volume of 0.32 M aqueous sucrose. The sample is then diluted 1:2 in 10 mM Na₂HPO₄/KH₂PO₄ buffer (pH = 7.4). A 100 µL sample of the buffered tissue suspension is incubated at 25°C for 60 minutes in the dark with 10 µL of test compound or vehicle for control and [³H] pirenzipine (0.5 nM, 0.04 µCi) q.s. 1 milliliter with the buffer solution. Atropine sulfate (2 µM) is added to half the samples being processed. Binding is terminated by vacuum filtration onto Whatman GF/B filters which are washed three times with the buffer solution (3 ml/wash, 4°C). The radioactivity of the filter-trapped material is determined by liquid scintillation spectroscopy and the IC₅₀ (50% inhibition of specific [³H] PZ binding) is calculated for the test compound. Specific [³H] PZ binding is defined as total binding minus binding in the presence of 2 µM atropine sulfate.

### EXAMPLE 24

The M2 receptor binding properties of the compounds of this invention were determined in the manner described for M1 receptor determinations with the following exceptions. Homogenized rat cerebellum tissue, diluted 1:3 in the above mentioned phosphate buffer, was employed for its M2 receptor sites and [³H] quinuclidinyl benzilate (0.23 nM, 0.01 µCi) was employed as the muscarinic receptor ligand. The concentration of atropine sulfate used in these experiments was 100 M. The assay tubes were incubated at 37°C for 60 minutes.

The muscarinic M2 receptor subtype serves to control presynaptic acetylcholine release. Activation of the M2 receptor inhibits acetycholine release, thereby exerting a negative influence on learning and memory processes which are at least partially regulated by the central cholinergic system. The muscarinic M1 receptor subtype is localized on the postsynaptic nerve cell where activation provides direct enhancement of the central cholinergic function. Enhancement of the central cholinergic function by direct stimulation of the M1 muscarinic receptor subtype provides one method of treatment for the central cholinergic dysfunction attending senile dementia of the Alzheimer type (SDAT) as a primary manifestation. Therefore, compounds to be used in the treatment of Alzheimer's disease and similar disease involving memory impairment and learning disabilities should exhibit selectivity for the M1 receptor over the M2 muscarinic receptor in the central nervous system. The compound produced in Example 15 epitomizes the desired selective property in this case.

In qualitatively evaluating the above data, high affinity values for 5-HT_{1A} receptors correlate (by analogy with buspirone) with anxiolytic/antidepressant activity, while lower values reflect a lesser activity. High affinity values for D₂ receptor binding (greater than 80%) begin to show some antipsychotic activity.

Hence, the compounds of this invention are antidepressant/anxiolytic agents useful in the treatment of depression and in alleviating anxiety and in the case of the products of Examples 3, 4, 6, 7, 14 and 15 they have some meaningful antipsychotic activity which is useful in the treatment of psychoses such as paranoia and schizophrenia. Central cholinergic activity is evidenced by the product of Example 15, which establishes the compounds as useful in the treatment of SDAT, Huntingdon's chorea, and the like diseases attending cholinergic hypofunction. As such, the compounds of this invention may be administered to a patient in need thereof, either neat or with a conventional pharmaceutical carrier. The pharmaceutical carrier may be solid or liquid as suitable for oral or parenteral administration.

**Table 1**

| Affinity for 5-HT_{1A}and 5-HT₂ Receptor Sites | | | | |
|---|---|---|---|---|
| Compound | % Inhibition at 1 µM or (Kᵢ, nM) | | % Inhibition at 100 nM | Affinity for D₂ Receptor Sites |
| | 5-HT_{1A} | 5-HT₂ | 5-HT_{1A} | D₂ |
| Example 1 | 98% (1 nM) | 92% | -- | 40% |
| Example 2 | 97% (1 nM) | 91% | -- | 40% |
| Example 3 | 97% | -- | -- | 93% |
| Example 4 | 100% | -- | -- | 91% |
| Example 5 | 83% | -- | -- | 62% |
| Example 6 | 94% | -- | -- | 87% |
| Example 7 | 91% | -- | -- | 95% |
| Example 8 | 94% | -- | -- | 32% |
| Example 9 | 93% | -- | -- | 27% |
| Example 10 | 82% | -- | -- | 6% |
| Example 11 | 91% | -- | -- | 40% |
| Example 12 | 98% | -- | -- | 70% |
| Example 13 | 97% | -- | -- | 67% |
| Example 14 | -- | -- | 100% | 100% |
| Example 15 | -- | -- | 99% | 94% |
| Example 16 | -- | -- | -- | -- |
| Example 17 | 88% (67 nM) | -- | -- | 27% |
| Example 18 | 99% | -- | -- | 72% |
| Example 19 | (20 nM) | -- | 75% | 31% |
| Gepirone | (65 nM) | 20% | (852 nM) | |
| Buspirone | 94% (10 nM) | 46% | (78 nM) | |

| Affinity for Muscarinic Acetylcholine Receptor Sites | | | | |
|---|---|---|---|---|
| Compound | IC₅₀for M₁ | IC₅₀for M₂ | M₂/M₁ Ratio | |
| Example 15 | 0.24 µM | 11 µM | 46 | |

In qualitatively evaluating the above data, high affinity values for 5-HT_{1A} receptors correlate (by analogy with gepirone) with anxiolytic-antidepressant activity, while lower values reflect a lesser activity. Affinity values for D₂ receptors indicate some antipsychotic activity.

Hence, the compounds of this invention are antidepressant, anxiolytic agents useful in the treatment of depression and in alleviating anxiety and in the case of the products of Examples 3, 4, 6 and 7, they have in addition to anxiolytic activity some meaningful antipsychotic activity which is useful in the treatment of psychoses such as paranoia and schizophrenia. As such, they may be administered to a patient in need thereof, either neat or with a conventional pharmaceutical carrier.

## Claims (Claims for the following Contracting State(s): AT, BE, CH, DE, FR, IT, LI, LU, NL, SE)

1. A compound having the formula 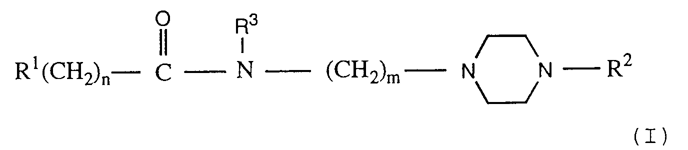 wherein R¹ is 1-adamantyl, 3-methyl-1-adamantyl, 3-noradamantyl, unsubstituted or substituted-2-indolyl, 3-indolyl, 2-benzofuranyl or 3-benzofuranyl wherein the substituents are selected from lower alkyl of 1 to 6 carbon atoms, lower alkoxy of 1 to 6 carbon atoms and halo; R² is unsubstituted or substituted phenyl, benzyl, pyridinyl, pyrimidinyl or pyrazinyl, wherein the substituents are selected from lower alkyl of 1 to 6 carbon atoms, lower alkoxy of 1 to 6 carbon atoms, trifluoromethyl and halo; R³ is H or lower alkyl of 1 to 3 carbon atoms; n is the integer 0 or 1; and m is an integer from 2 to 5; or a pharmaceutically acceptable salt thereof, with the proviso that when R¹ is substituted or unsubstituted 2-benzofuranyl, and n is 0 then R² is other than phenyl or phenyl substituted by halogen, alkyl of 1 to 4 carbon atoms alkoxy of 1 to 4 carbon atoms or trifluoromethyl.

2. A compound according to Claim 1 wherein R² is phenyl, methoxyphenyl, chlorophenyl, 3-(trifluoromethyl)phenyl, pyridinyl or pyrimidinyl.

3. A compound according to Claims 1 or Claim 2 wherein m is 2 or 3.

4. A compound according to Claim 1 wherein R¹ is 1-adamantyl, 3-methyl-1-adamantyl, 2-indolyl, 2-benzofuranyl; R² is unsubstituted or substituted phenyl, wherein the substituents are selected form methoxy and chloro, pyridinyl or 2-pyrimidinyl; R³ is H; n is the integer 0 or 1; m is the integer 2 or 3 or a pharmaceutically acceptable salt thereof.

5. A compound as claimed in Claim 1 which is one of the following:
N-[3-[4-(2-methoxyphenyl)-1-piperazinyl]propyl]tricyclo[3.3.1.1^{3,7}]decane-1-carboxamide;
N-[2-[4-(2-pyrimidyl)-1-piperazinyl]ethyl]tricyclo[3.3.1.1^{3,7}]decane-1-carboxamide;
N-[2-[4-(2-methoxyphenyl)-1-piperazinyl]ethyl]tricyclo[3.3.1.1^{3,7}]decane-1-carboxamide;
N-[2-[4-(3-chlorophenyl)-1-piperazinyl]ethyl]tricyclo[3.3.1.1.^{3,7}]-decane-1-carboxamide;
N-[2-[4-(2-pyrimidyl)-1-piperazinyl]ethyl]benzofurane-2-carboxamide;
N-[3-[4-(2-methoxyphenyl)-1-piperazinyl]propyl]-(1H)-indole-2-carboxamide;
N-[3-[4-(3-chlorophenyl)-1-piperazinyl]propyl]tricyclo[3.3.1.1^{3,7}]decane-1-carboxamide;.
N-[2-[4-(2-pyrimidyl)-1- piperazinyl]ethyl]-3-methyltricyclo[3.3.1.1^{3,7}]decane-1-acetic acid carboxamide;
N-[3-[4-(2-pyrimidyl)-1-piperazinyl]propyl]tricyclo-[3.3.1.1^{3,7}]decane-1-carboxamide;
N-[2-[4-(3-chlorophenyl)-1-piperazinyl]ethyl]-3-methyltricyclo[3.3.1.1^{3,7}]decane-1-acetic acid carboxamide;
N-[2-[4-(2-methoxyphenyl)-1-piperazinyl]ethyl]-3-methyltricyclo[3.3.1.1^{3,7}]decane-1-acetic acid carboxamide;
N-[2-[4-(2-pyrimidyl)-1-piperazinyl]ethyl]hexahydro-2,5-methanopentalene-3a-(1H)-carboxamide;
N-[2-[4-(2-methoxyphenyl)-1-piperazinyl]ethyl]hexahydro-2,5-methanopentalene-3a(1H)-carboxamide;
N-[2-[4-(3-chlorophenyl)-1-piperazinyl]ethyl]hexahydro-2,5-methanopentalene-3a(1H)-carboxamide;
N-[2-[4-[3-(trifluoromethyl)-phenyl]-1-piperazinyl]ethyl]hexahydro-2,5-methanepentalene-3a(1H)-carboxamide;
N-[3-[4-(2-pyrimidyl)-1-piperazinyl]propyl]-hexahydro-2,5-methanopentalene-3a(1H)-carboxamide; or
N-[3-[4-(2-methoxyphenyl)-1-piperazinyl]propyl]hexahydro-2,5-methanopentalene-3a(1H)-carboxamide; or a pharmaceutically acceptable salt thereof.

6. A pharmaceutical composition which comprises a compound of formula I, or a pharmaceutically acceptable salt thereof, as defined in Claim 1, in association with a pharmaceutically acceptable carrier.

7. A process for preparing a compound of formula I as defined in Claim 1 or a pharmaceutically acceptable salt thereof which comprises one of the following:
(a) acylating a compound of formula 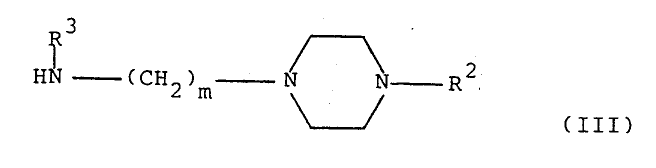 with an acylating agent containing the group R⁵ wherein R⁵ is:
R¹-(CH₂)ₙC(O)- (IIa)
wherein n, m, R¹, R², R³ are as defined in Claim 1 to give a compound of formula (I);
or
(b) a cyclic amine having the formula (IV) 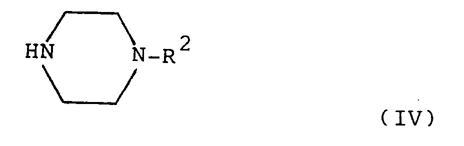 wherein R² is as defined in Claim 1 or a salt thereof is alkylated to introduce the substituted alkyl group having the formula (V) 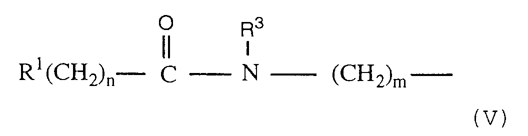 by reaction with a compound having the formula R⁴-Y, wherein R⁴ is the group having formula (V) and Y is a leaving group,
or
(c) a cyclic amine having the formula (IV) as defined above or a salt thereof is subjected to reductive alkylation with an aldehyde having the formula (VI) 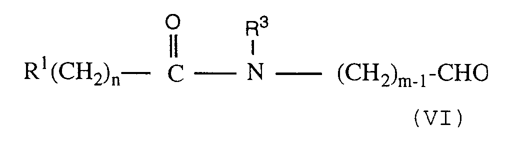 wherein n, R¹, R³ and m are as defined in Claim 1
or
(d) a compound having the formula (I) is converted into an acid addition salt thereof by addition of an acid or an acid addition salt of a compound having formula (I) is subjected to neutralization to form the compound having formula (I).

8. A process for preparing a compound having the formula I as defined in Claim 1, or a pharmaceutically acceptable salt thereof which comprises reacting a carboxylic acid halide of formula (II) 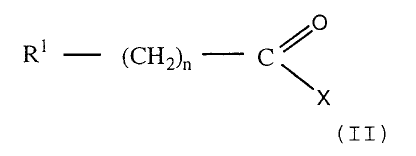 wherein R¹ and n are as defined in Claim 1 and X is a halide with an aminoalkyl piperazine of formula (III) 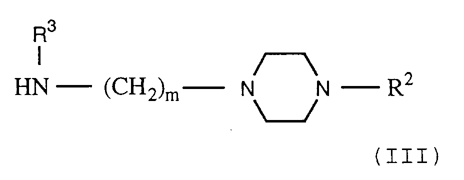 wherein R², R³ and m are as defined above.

9. A compound of formula I as claimed in Claim 1 for use as a pharmaceutical.

10. Use of a compound having the formula 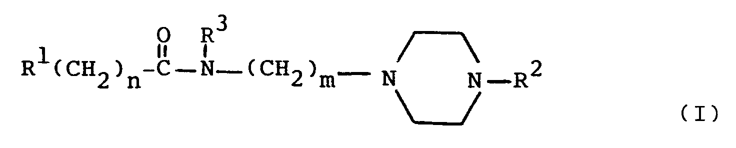 wherein R¹ is 1-adamantyl, 3-methyl-1-adamantyl, 3-noradamantyl, unsubstituted or substituted 2-indolyl, 3-indolyl, 2-benzofuranyl or 3-benzofuranyl wherein the substituents are selected from lower alkyl of 1 to 6 carbon atoms, lower alkoxy or 1 to 6 carbon atoms and halo; R² is unsubstituted or substituted phenyl, benzyl, pyridinyl, pyrimidinyl or pyrazinyl, wherein the substituents are selected from lower alkyl of 1 to 6 carbon atoms, lower alkoxy or 1 to 6 carbon atoms, trifluoromethyl and halo; R³ is H or lower alkyl of 1 to 3 carbon atoms; n is the integer 0 or 1; and m is an integer from 2 to 5; or a pharmaceutically acceptable salt thereof, for the manufacture of a medicament for the treatment of anxiety, depression or psychoses.

11. Use as claimed in Claim 10 in which the compound of formula I is
N-[3-[4-(2-methoxyphenyl)-1-piperazinyl]propyl]benzofurane-2-carboxamide or a pharmaceutically acceptable salt thereof.

## Claims (Claims for the following Contracting State(s): ES, GR)

1. A process for preparing a compound having the formula 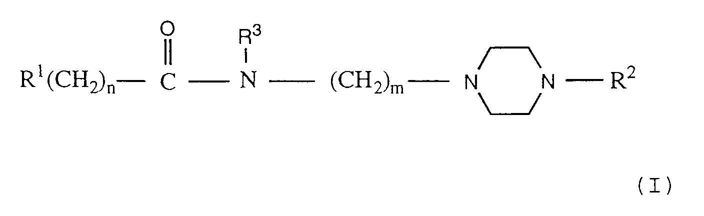 wherein R¹ is 1-adamantyl, 3-methyl-1-adamantyl, 3-noradamantyl, unsubstituted or substituted-2-indolyl, 3-indolyl, 2-benzofuranyl or 3-benzofuranyl wherein the substituents are selected from lower alkyl of 1 to 6 carbon atoms, lower alkoxy of 1 to 6 carbon atoms and halo; R² is unsubstituted or substituted phenyl, benzyl, pyridinyl, pyrimidinyl or pyrazinyl, wherein the substituents are selected from lower alkyl of 1 to 6 carbon atoms, lower alkoxy of 1 to 6 carbon atoms, trifluoromethyl and halo; R³ is H or lower alkyl of 1 to 3 carbon atoms; n is the integer 0 or 1; and m is an integer from 2 to 5; or a pharmaceutically acceptable salt thereof, with the proviso that when R¹ is substituted or unsubstituted 2-benzofuranyl, and n is 0 then R² is other than phenyl or phenyl substituted by halogen, alkyl of 1 to 4 carbon atoms, alkoxy of 1 to 4 carbon atoms or trifluoromethyl, which comprises one of the following:
(a) acylating a compound of formula 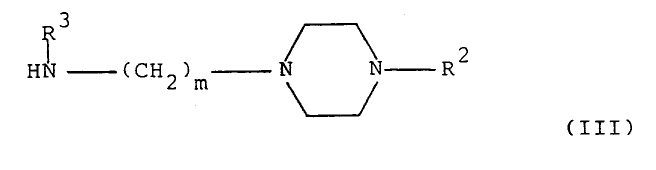 with an acylating agent containing the group R⁵ wherein R⁵ is:
R¹-(CH₂)ₙC(O)- (IIa)
wherein n, m, R¹, R², R³ are as defined above to give a compound of formula (I);
or
(b) a cyclic amine having the formula (IV) 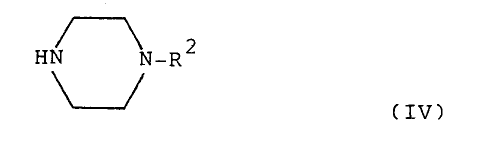 wherein R² is as defined above or a salt thereof is alkylated to introduce the substituted alkyl group having the formula (V) 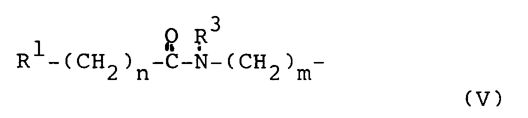 by reaction with a compound having the formula R⁴-Y, wherein R⁴ is the group having formula (V) and Y is a leaving group,
or
(c) a cyclic amine having the formula (IV) as defined above or a salt thereof is subjected to reductive alkylation with an aldehyde having the formula (VI) 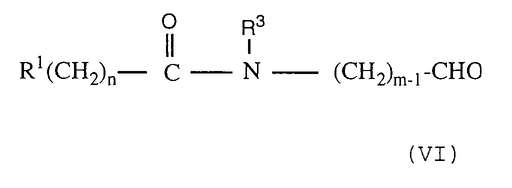 wherein n, R¹, R³ and m are as defined above;
or
(d) a compound having the formula (I) is converted into an acid addition salt thereof by addition of an acid or an acid addition salt of a compound having formula (I) is subjected to neutralization to form the compound having formula (I).

2. A process for preparing a compound having the formula I as defined in Claim 1, or a pharmaceutically acceptable salt thereof which comprises reacting a carboxylic acid halide of formula (II) 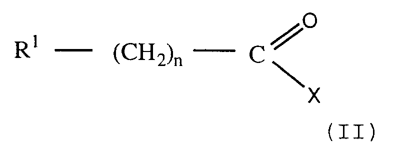 wherein R¹ and n are as defined above and X is halide with an aminoalkyl piperazine of formula (III) 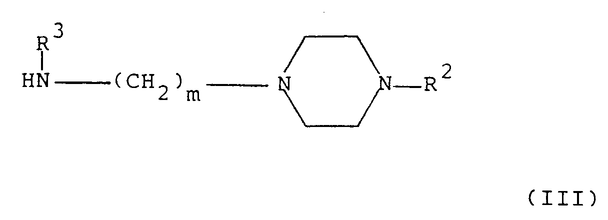 wherein R², R³ and m are as defined above.

3. A process according to Claim 1 or Claim 2 wherein R² is phenyl, methoxyphenyl, chlorophenyl, 3-(trifluoromethyl)phenyl, pyridinyl or pyrimidinyl.

4. A process according to any one of Claims 1 to 3 wherein m is 2 or 3.

5. A process according to Claim 1 or Claim 2 wherein R¹ is 1-adamantyl, 3-methyl-1-adamantyl, 2-indolyl, 2-benzofuranyl; R² is unsubstituted or substituted phenyl, wherein the substituents are selected form methoxy and chloro, pyridinyl or 2-pyrimidinyl; R³ is H; n is the integer 0 or 1; m is the integer 2 or 3 or a pharmaceutically acceptable salt thereof.

6. A process as claimed in Claim 1 or Claim 2 in which the product is N-[3-[4-(2-methoxyphenyl)-1-piperazinyl]propyl]tricyclo[3.3.1.1^{3,7}]decane-1-carboxamide;
N-[2-[4-(2-pyrimidyl)-1-piperazinyl]ethyl]tricyclo[3.3.1.1^{3,7}]decane-1-carboxamide;
N-[2-[4-(2-methoxvphenyl)-1-piperazinyl]ethyl)tricyclo[3.3.1.1^{3,7}]decane-1-carboxamide;
N-[2-[4-(3-chlorophenyl)-1-piperazinyl]ethyl]tricyclo[3.3.1.1.^{3,7}]- decane-1-carboxamide;
N-[2-[4-(2-pyrimidyl)-1-piperazinyl)ethyl]benzofurane-2-carboxamide;
N-[3-[4-(2-methoxyphenyl)-1-piperazinyl]propyl]-(1H)-indole-2-carboxamide;
N-[3-[4-(3-chlorophenyl)-1-piperazinyl]propyl]tricyclo[3.3.1.1^{3,7}]decane-1-carboxamide;
N-[2-[4-(2-pyrimidyl)-1- piperazinyl]ethyl]-3-methyltricyclo[3.3.1.1^{3,7}]decane-1-acetic acid carboxamide;
N-[3-[4-(2-pyrimidyl)-1-piperazinyl]propyl]tricyclo-[3.3.1.1^{3,7}]decane-1-carboxamide;
N-[2-[4-(3-chlorophenyl)-1-piperazinyl]ethyl]-3-methyltricyclo[3.3.1.1^{3,7}]decane-1-acetic acid carboxamide;
N-[2-[4-(2-methoxyphenyl)-1-piperazinyl]ethyl]-3-methyltricyclo[3.3.1.1^{3,7}]decane-1-acetic acid carboxamide;
N-[2-[4-(2-pyrimidyl)-1-piperazinyl]ethyl]hexahydro-2,5-methanopentalene-3a-(1H)-carboxamide;
N-[2-[4-(2-methoxyphenyl)-1-piperazinyl]ethyl]hexahydro-2,5-methanopentalene-3a(1H)-carboxamide;
N-[2-[4-(3-chlorophenyl)-1-piperazinyl]ethyl]hexahydro-2,5-methanopentalene-3a(1H)-carboxamide;
N-[2-[4-[3-(trifluoromethyl)-phenyl]-1-piperazinyl]ethyl]hexahydro-2,5-methanepentalene-3a(1H)-carboxamide;
N-[3-[4-(2-pyrimidyl)-1-piperazinyl]propyl]-hexahydro-2,5-methanopentalene-3a(1H)-carboxamide;
N-[3-[4-(2-methoxyphenyl)-1-piperazinyl]propyl]hexahydro-2,5-methanopentalene-3a(1H)-carboxamide; or a pharmaceutically acceptable salt thereof.

7. A process for preparing a pharmaceutical composition which comprises bringing a compound of formula I, or a pharmaceutically acceptable salt thereof, as defined in Claim 1, into association with a pharmaceutically acceptable carrier.

8. Use of a compound having the formula 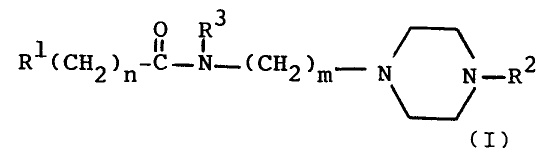 wherein R¹ is 1-adamantyl, 3-methyl-1-adamantyl, 3-noradamantyl, unsubstituted or substituted 2-indolyl, 3-indolyl, 2-benzofuranyl or 3-benzofuranyl wherein the substituents are selected from lower alkyl of 1 to 6 carbon atoms, lower alkoxy or 1 to 6 carbon atoms and halo; R² is unsubstituted or substituted phenyl, benzyl, pyridinyl, pyrimidinyl or pyrazinyl, wherein the substituents are selected from lower alkyl of 1 to 6 carbon atoms, lower alkoxy or 1 to 6 carbon atoms, trifluoromethyl and halo; R³ is H or lower alkyl of 1 to 3 carbon atoms; n is the integer 0 or 1; and m is an integer from 2 to 5; or a pharmaceutically acceptable salt thereof, for the manufacture of a medicament for the treatment of anxiety, depression or psychoses.

9. Use as claimed in Claim 8 in which the compound of formula I is
N-[3-[4-(2-methoxyphenyl)-1-piperazinyl]propyl]benzofurane-2-carboxamide or a pharmaceutically acceptable salt thereof.

## Patentansprüche (Patentansprüche für folgende(n) Vertragsstaat(en): AT, BE, CH, DE, FR, IT, LI, LU, NL, SE)

1. Verbindung der Formel 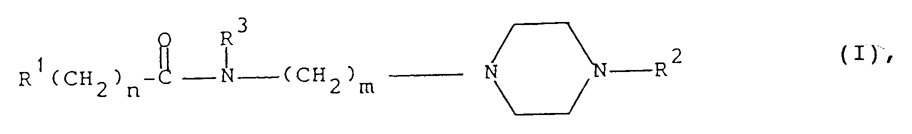 worin R¹ 1-Adamantyl, 3-Methyl-1-adamantyl, 3-Noradamantyl, unsubstituiertes oder substituiertes 2-Indolyl, 3-Indolyl, 2-Benzofuranyl oder 3-Benzofuranyl ist, wobei die Substituenten ausgewählt sind aus nied.Alkyl mit 1 bis 6 Kohlenstoffatomen, nied.Alkoxy mit 1 bis 6 Kohlenstoffatomen und Halogen; R² unsubstituiertes oder substituiertes Phenyl, Benzyl, Pyridinyl, Pyrimidinyl oder Pyrazinyl darstellt, wobei die Substituenten ausgewählt sind aus nied.Alkyl mit 1 bis 6 Kohlenstoffatomen, nied.Alkoxy mit 1 bis 6 Kohlenstoffatomen, Trifluormethyl und Halogen; R³ die Bedeutung H oder nied.Alkyl mit 1 bis 3 Kohlenstoffatomen hat; n die ganze Zahl Null oder 1 ist; und m eine ganze Zahl von 2 bis 5 ist; oder ein pharmazeutisch annehmbares Salz hievon, mit der Maßgabe, daß, wenn R¹ substituiertes oder unsubstituiertes 2-Benzofuranyl darstellt, und n Null ist, R² von Phenyl oder durch Halogen, Alkyl mit 1 bis 4 Kohlenstoffatomen, Alkoxy mit 1 bis 4 Kohlenstoffatomen oder Trifluormethyl substituiertem Phenyl verschieden ist.

2. Verbindung nach Anspruch 1, worin R² Phenyl, Methoxyphenyl, Chlorphenyl, 3-(Trifluormethyl)-phenyl, Pyridinyl oder Pyrimidinyl bedeutet.

3. Verbindung nach Anspruch 1 oder 2, worin m 2 oder 3 ist.

4. Verbindung nach Anspruch 1, worin R¹ 1-Adamantyl, 3-Methyl-1-adamantyl, 2-Indolyl oder 2-Benzofuranyl darstellt; R² unsubstituiertes oder substituiertes Phenyl ist, wobei die Substituenten ausgewählt sind aus Methoxy und Chlor, Pyridinyl oder 2-Pyridinyl; R³ die Bedeutung H hat; n die ganze Zahl Null oder 1 ist; und m die ganze Zahl 2 oder 3 ist; oder ein pharmazeutisch annehmbares Salz hievon.

5. Verbindung nach Anspruch 1, nämlich:
N-[3-[4-(2-Methoxyphenyl)-1-piperazinyl]-propyl]-tricyclo-[3.3.1.1^{3,7}]decan-1-carboxamid;
N-[2-[4-(2-Pyrimidyl)-1-piperazinyl]-ethyl]-tricyclo-[3.3.1.1^{3,7}]decan-1-carboxamid;
N-[2-[4-(2-Methoxyphenyl)-1-piperazinyl]-ethyl]-tricyclo-[3.3.1.1^{3,7}]decan-1-carboxamid;
N-[2-[4-(3-Chlorphenyl)-1-piperazinyl]-ethyl]-tricyclo-[3.3.1.1^{3,7}]decan-1-carboxamid;
N-[2-[4-(2-Pyrimidyl)-1-piperazinyl]-ethyl]-benzofuran-2-carboxamid;
N-[3-[4-(2-Methoxyphenyl)-1-piperazinyl]-propyl]-(1H)-indol-2-carboxamid;
N-[3-[4-(3-Chlorphenyl)-1-piperazinyl]-propyl]-tricyclo-[3.3.1.1^{3,7}]decan-1-carboxamid;
N-[2-[4-(2-Pyrimidyl)-1-piperazinyl]-ethyl]-3-methyltricyclo-[3.3.1.1^{3,7}]decan-1-essigsäurecarboxamid;
N-[3-[4-(2-Pyrimidyl)-1-piperazinyl]-propyl]-tricyclo-[3.3.1.1^{3,7}]decan-1-carboxamid;
N-[2-[4-(3-Chlorphenyl)-1-piperazinyl]-ethyl]-3-methyltricyclo-[3.3.1.1^{3,7}]decan-1-essigsäurecarboxamid;
N-[2-[4-(2-Methoxyphenyl)-1-piperazinyl]-ethyl]-3-methyltricyclo[3.3.1.1^{3,7}]decan-1-essigsäurecarboxamid;
N-[2-[4-(2-Pyrimidyl)-1-piperazinyl]-ethyl]-hexahydro-2,5-methanopentalen-3a(1H)-carboxamid;
N-[2-[4-(2-Methoxyphenyl)-1-piperazinyl]-ethyl]-hexahydro-2,5-methanopentalen-3a(1H)-carboxamid;
N-[2-[4-(3-Chlorphenyl)-1-piperazinyl]-ethyl]-hexahydro-2,5-methanopentalen-3a(1H)-carboxamid;
N-[2-[4-[3-(Trifluormethyl)-phenyl]-1-piperazinyl]-ethyl]-hexahydro-2,5-methanopentalen-3a(1H)-carboxamid;
N-[3-[4-(2-Pyrimidyl)-1-piperazinyl]-propyl]-hexahydro-2,5-methanopentalen-3a(1H)-carboxamid; oder
N-[3-[4-(2-Methoxyphenyl)-1-piperazinyl]-propyl]-hexahydro-2,5-methanopentalen-3a(1H)-carboxamid;
oder ein pharmazeutisch annehmbares Salz hievon.

6. Pharmazeutische Zusammensetzung, welche eine Verbindung der Formel (I), oder ein pharmazeutisch annehmbares Salz hievon, wie in Anspruch 1 definiert, in Vereinigung mit einem pharmazeutisch annehmbaren Träger umfaßt.

7. Verfahren zur Herstellung einer Verbindung der Formel (I), wie in Anspruch 1 definiert, oder eines pharmazeutisch annehmbaren Salzes hievon, welches umfaßt, daß:
(a) eine Verbindung der Formel 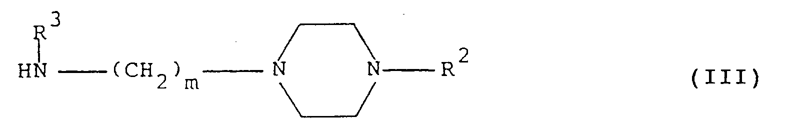 mit einem Acylierungsmittel, das die Gruppe R⁵ enthält, wobei R⁵ die Bedeutung hat:
R¹-(CH₂)ₙC(O)- (IIa),
worin n, m, R¹, R² und R³ wie in Anspruch 1 definiert sind, acyliert wird, wobei eine Verbindung der Formel (I) erhalten wird; oder
(b) ein cyclisches Amin der Formel (IV) 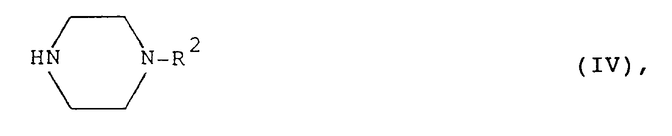 worin R² wie in Anspruch 1 definiert ist, oder ein Salz hievon, alkyliert wird, wobei die substituierte Alkylgruppe der Formel (V) 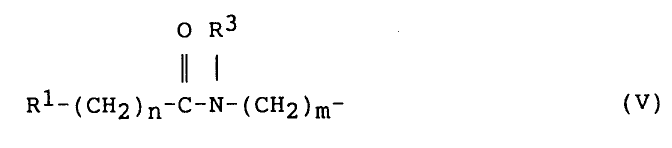 durch das Umsetzen mit einer Verbindung der Formel R⁴-Y, worin R⁴ eine Gruppe der Formel (V) ist, und Y eine Abgangsgruppe darstellt, eingeführt wird, oder
(c) ein cyclisches Amin der Formel (IV), wie oben definiert, oder ein Salz hievon, einer reduktiven Alkylierung mit einem Aldehyd der Formel (VI) 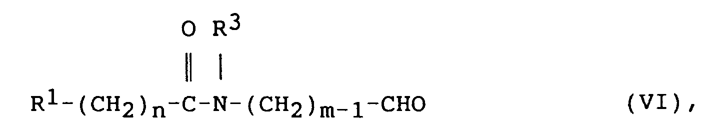 worin n, R¹, R³ und m wie in Anspruch 1 definiert sind, unterworfen wird, oder
(d) eine Verbindung der Formel (I) in ein Säureadditionssalz hievon übergeführt wird, indem eine Säure oder ein Säureadditionssalz einer Verbindung der Formel (I) einer Neutralisation ausgesetzt wird, wobei die Verbindung der Formel (I) erhalten wird.

8. Verfahren zur Herstellung einer Verbindung der Formel (I), wie in Anspruch 1 definiert, oder eines pharmazeutisch annehmbaren Salzes hievon, welches umfaßt: Umsetzen eines Carbonsäurehalogenids der Formel (II) 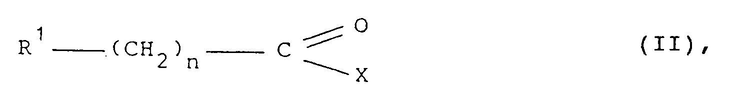 worin R¹ und n wie in Anspruch 1 definiert sind, und X ein Halogenid darstellt, mit einem Aminoalkylpiperazin der Formel (III) 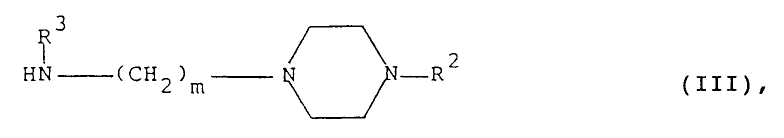 worin R², R³ und m wie oben definiert sind.

9. Verbindung der Formel (I) nach Anspruch 1 zur Verwendung als Pharmazeutikum.

10. Verwendung einer Verbindung der Formel 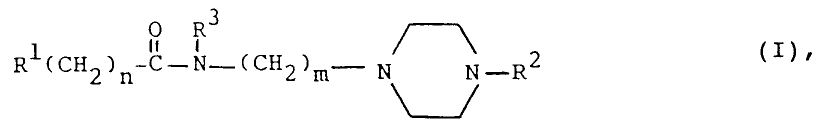 worin R¹ 1-Adamantyl, 3-Methyl-1-adamantyl, 3-Noradamantyl, unsubstituiertes oder substituiertes 2-Indolyl, 3-Indolyl, 2-Benzofuranyl oder 3-Benzofuranyl ist, wobei die Substituenten ausgewählt sind aus nied.Alkyl mit 1 bis 6 Kohlenstoffatomen, nied.Alkoxy mit 1 bis 6 Kohlenstoffatomen und Halogen; R² unsubstituiertes oder substituiertes Phenyl, Benzyl, Pyridinyl, Pyrimidinyl oder Pyrazinyl darstellt, wobei die Substituenten ausgewählt sind aus nied.Alkyl mit 1 bis 6 Kohlenstoffatomen, nied.Alkoxy mit 1 bis 6 Kohlenstoffatomen, Trifluormethyl und Halogen; R³ die Bedeutung H oder nied.Alkyl mit 1 bis 3 Kohlenstoffatomen hat; n die ganze Zahl Null oder 1 ist; und m eine ganze Zahl von 2 bis 5 ist; oder eines pharmazeutisch annehmbaren Salzes hievon, bei der Herstellung eines Medikaments zur Behandlung von Angstzuständen, Depressionen oder Psychosen.

11. Verwendung nach Anspruch 10, wobei die Verbindung der Formel (I)
N-[3-[4-(2-Methoxyphenyl)-1-piperazinyl]-propyl]-benzofuran-2-carboxamid
oder ein pharmazeutisch annehmbares Salz hievon ist.

## Patentansprüche (Patentansprüche für folgende(n) Vertragsstaat(en): ES, GR)

1. Verfahren zur Herstellung einer Verbindung der Formel 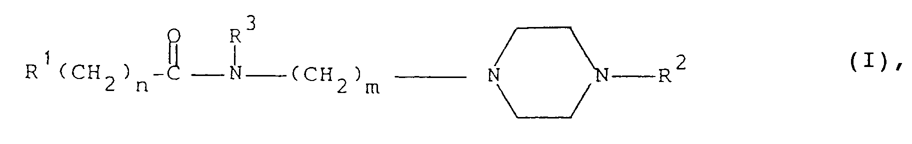 worin R¹ 1-Adamantyl, 3-Methyl-1-adamantyl, 3-Noradamantyl, unsubstituiertes oder substituiertes 2-Indolyl, 3-Indolyl, 2-Benzofuranyl oder 3-Benzofuranyl ist, wobei die Substituenten ausgewählt sind aus nied.Alkyl mit 1 bis 6 Kohlenstoffatomen, nied.Alkoxy mit 1 bis 6 Kohlenstoffatomen und Halogen; R² unsubstituiertes oder substituiertes Phenyl, Benzyl, Pyridinyl, Pyrimidinyl oder Pyrazinyl darstellt, wobei die Substituenten ausgewählt sind aus nied.Alkyl mit 1 bis 6 Kohlenstoffatomen, nied.Alkoxy mit 1 bis 6 Kohlenstoffatomen, Trifluormethyl und Halogen; R³ die Bedeutung H oder nied.Alkyl mit 1 bis 3 Kohlenstoffatomen hat; n die ganze Zahl Null oder 1 ist; und m eine ganze Zahl von 2 bis 5 ist; oder eines pharmazeutisch annehmbaren Salzes hievon, mit der Maßgabe, daß, wenn R¹ substituiertes oder unsubstituiertes 2-Benzofuranyl darstellt, und n Null ist, R² von Phenyl oder durch Halogen, Alkyl mit 1 bis 4 Kohlenstoffatomen, Alkoxy mit 1 bis 4 Kohlenstoffatomen oder Trifluormethyl substituiertem Phenyl verschieden ist, welches Verfahren umfaßt, daß:
(a) eine Verbindung der Formel 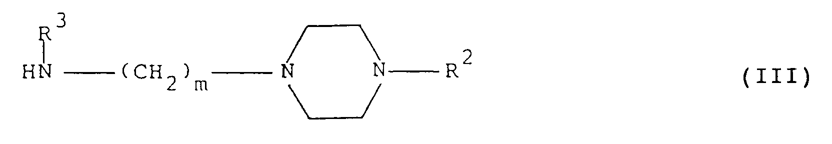 mit einem Acylierungsmittel, das die Gruppe R⁵ enthält, wobei R⁵ die Bedeutung hat:
R¹-(CH₂)ₙC(O)- (IIa),
worin n, m, R¹, R² und R³ wie oben definiert sind, acyliert wird, wobei eine Verbindung der Formel (I) erhalten wird; oder
(b) ein cyclisches Amin der Formel (IV) 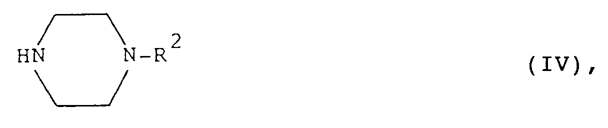 worin R² wie oben definiert ist, oder ein Salz hievon, alkyliert wird, wobei die substituierte Alkylgruppe der Formel (V) 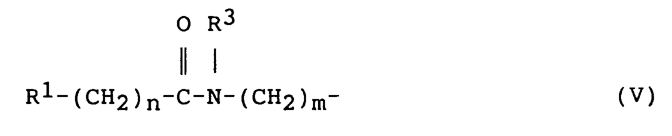 durch das Umsetzen mit einer Verbindung der Formel R⁴-Y, worin R⁴ eine Gruppe der Formel (V) ist, und Y eine Abgangsgruppe darstellt, eingeführt wird, oder
(c) ein cyclisches Amin der Formel (IV), wie oben definiert, oder ein Salz hievon, einer reduktiven Alkylierung mit einem Aldehyd der Formel (VI) 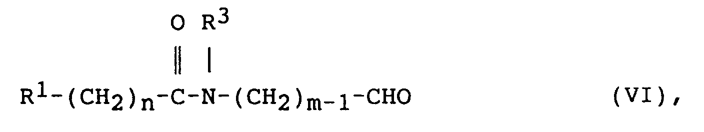 worin n, R¹, R³ und m wie in Anspruch 1 definiert sind, unterworfen wird, oder
(d) eine Verbindung der Formel (I) in ein Säureadditionssalz hievon übergeführt wird, indem eine Säure oder ein Säureadditionssalz einer Verbindung der Formel (I) einer Neutralisation ausgesetzt wird, wobei die Verbindung der Formel (I) erhalten wird.

2. Verfahren zur Herstellung einer Verbindung der Formel (I), wie in Anspruch 1 definiert, oder eines pharmazeutisch annehmbaren Salzes hievon, welches umfaßt: Umsetzen eines Carbonsäurehalogenids der Formel (II) 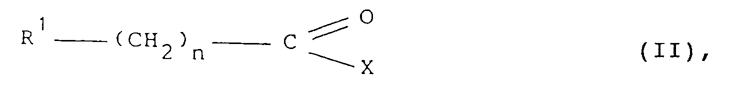 worin R¹ und n wie oben definiert sind, und X ein Halogenid darstellt, mit einem Aminoalkylpiperazin der Formel (III) 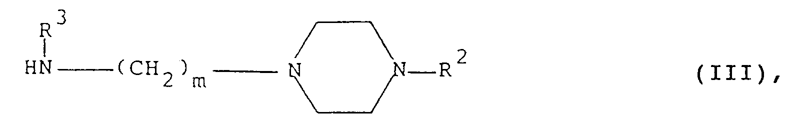 worin R², R³ und m wie oben definiert sind.

3. Verfahren nach Anspruch 1 oder 2, wobei R² Phenyl, Methoxyphenyl, Chlorphenyl, 3-(Trifluormethyl)-phenyl, Pyridinyl oder Pyrimidinyl bedeutet.

4. Verfahren nach einem der Ansprüche 1 bis 3, wobei m 2 oder 3 ist.

5. Verfahren nach Anspruch 1 oder 2, wobei R¹ 1-Adamantyl, 3-Methyl-1-adamantyl, 2-Indolyl oder 2-Benzofuranyl darstellt; R² unsubstituiertes oder substituiertes Phenyl ist, wobei die Substituenten ausgewählt sind aus Methoxy und Chlor, Pyridinyl oder 2-Pyridinyl; R³ die Bedeutung H hat; n die ganze Zahl Null oder 1 ist; und m die ganze Zahl 2 oder 3 ist; oder eines pharmazeutisch annehmbaren Salzes hievon.

6. Verfahren nach Anspruch 1 oder 2, bei welchem das Produkt:
N-[3-[4-(2-Methoxyphenyl)-1-piperazinyl]-propyl]-tricyclo-[3.3.1.1^{3,7}]decan-1-carboxamid;
N-[2-[4-(2-Pyrimidyl)-1-piperazinyl]-ethyl]-tricyclo-[3.3.1.1^{3,7}]decan-1-carboxamid;
N-[2-[4-(2-Methoxyphenyl)-1-piperazinyl]-ethyl]-tricyclo-[3.3.1.1^{3,7}]decan-1-carboxamid;
N-[2-[4-(3-Chlorphenyl)-1-piperazinyl]-ethyl]-tricyclo-[3.3.1.1^{3,7}]decan-1-carboxamid;
N-[2-[4-(2-Pyrimidyl)-1-piperazinyl]-ethyl]-benzofuran-2-carboxamid;
N-[3-[4-(2-Methoxyphenyl)-1-piperazinyl]-propyl]-(1H)-indol-2-carboxamid;
N-[3-[4-(3-Chlorphenyl)-1-piperazinyl]-propyl]-tricyclo-[3.3.1.1^{3,7}]decan-1-carboxamid;
N-[2-[4-(2-Pyrimidyl)-1-piperazinyl]-ethyl]-3-methyltricyclo-[3.3.1.1^{3,7}]decan-1-essigsäurecarboxamid;
N-[3-[4-(2-Pyrimidyl)-1-piperazinyl]-propyl]-tricyclo-[3.3.1.1^{3,7}]decan-1-carboxamid;
N-[2-[4-(3-Chlorphenyl)-1-piperazinyl]-ethyl]-3-methyltricyclo-[3.3.1.1^{3,7}]decan-1-essigsäurecarboxamid;
N-[2-[4-(2-Methoxyphenyl)-1-piperazinyl]-ethyl]-3-methyltricyclo[3.3.1.1^{3,7}]decan-1-essigsäurecarboxamid;
N-[2-[4-(2-Pyrimidyl)-1-piperazinyl]-ethyl]-hexahydro-2,5-methanopentalen-3a(1H)-carboxamid;
N-[2-[4-(2-Methoxyphenyl)-1-piperazinyl]-ethyl]-hexahydro-2,5-methanopentalen-3a(1H)-carboxamid;
N-[2-[4-(3-Chlorphenyl)-1-piperazinyl]-ethyl]-hexahydro-2,5-methanopentalen-3a(1H)-carboxamid;
N-[2-[4-[3-(Trifluormethyl)-phenyl]-1-piperazinyl]-ethyl]-hexahydro-2,5-methanopentalen-3a(1H)-carboxamid;
N-[3-[4-(2-Pyrimidyl)-1-piperazinyl]-propyl]-hexahydro-2,5-methanopentalen-3a(1H)-carboxamid; oder
N-[3-[4-(2-Methoxyphenyl)-1-piperazinyl]-propyl]-hexahydro-2,5-methanopentalen-3a(1H)-carboxamid;
oder ein pharmazeutisch annehmbares Salz hievon ist.

7. Verfahren zur Herstellung einer pharmazeutischen Zusammensetzung, welches das Vereinigen einer Verbindung der Formel (I), oder eines pharmazeutisch annehmbaren Salzes hievon, wie in Anspruch 1 definiert, mit einem pharmazeutisch annehmbaren Träger umfaßt.

8. Verwendung einer Verbindung der Formel 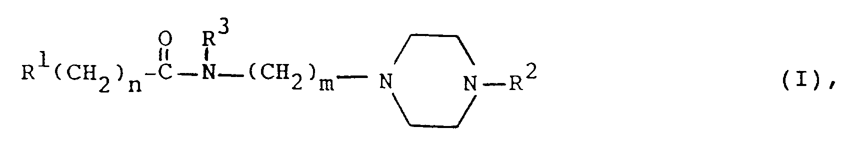 worin R¹ 1-Adamantyl, 3-Methyl-1-adamantyl, 3-Noradamantyl, unsubstituiertes oder substituiertes 2-Indolyl, 3-Indolyl, 2-Benzofuranyl oder 3-Benzofuranyl ist, wobei die Substituenten ausgewählt sind aus nied.Alkyl mit 1 bis 6 Kohlenstoffatomen, nied.Alkoxy mit 1 bis 6 Kohlenstoffatomen und Halogen; R² unsubstituiertes oder substituiertes Phenyl, Benzyl, Pyridinyl, Pyrimidinyl oder Pyrazinyl darstellt, wobei die Substituenten ausgewählt sind aus nied.Alkyl mit 1 bis 6 Kohlenstoffatomen, nied.Alkoxy mit 1 bis 6 Kohlenstoffatomen, Trifluormethyl und Halogen; R³ die Bedeutung H oder nied.Alkyl mit 1 bis 3 Kohlenstoffatomen hat; n die ganze Zahl Null oder 1 ist; und m eine ganze Zahl von 2 bis 5 ist; oder eines pharmazeutisch annehmbaren Salzes hievon, bei der Herstellung eines Medikaments zur Behandlung von Angstzuständen, Depressionen oder Psychosen.

9. Verwendung nach Anspruch 8, wobei die Verbindung der Formel (I)
N-[3-[4-(2-Methoxyphenyl)-1-piperazinyl]-propyl]-benzofuran-2-carboxamid
oder ein pharmazeutisch annehmbares Salz hievon ist.

## Revendications (Revendications pour l'(les) Etat(s) contractant(s) suivant(s): AT, BE, CH, DE, FR, IT, LI, LU, NL, SE)

1. Composé ayant la formule : 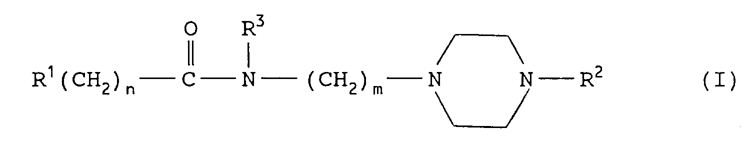 où :
R¹ est 1-adamantyle, 3-méthyl-1-adamantyle, 3-noradamantyle, 2-indolyle, 3-indolyle, 2-benzofurannyle ou 3-benzofurannyle substitué ou non substitué, où les substituants sont choisis parmi alcoyle inférieur en 1 à 6 atomes de carbone, alcoxy inférieur en 1 à 6 atomes de carbone et halo;
R² est phényle, benzyle, pyridinyle, pyrimidinyle ou pyrazinyle substitué ou non substitué, où les substituants sont choisis parmi alcoyle inférieur en 1 à 6 atomes de carbone, alcoxy inférieur en 1 à 6 atomes de carbone, trifluorométhyle et halo;
R³ est H ou alcoyle inférieur en 1 à 3 atomes de carbone;
n est l'entier 0 ou 1, et
m est un entier de 2 à 5, ou
un sel pharmaceutiquement acceptable de celui-ci, à la condition que, lorsque R¹ est 2-benzofurannyle substitué ou non substitué et lorsque n est 0, alors R² est autre que phényle ou phényle substitué par halogène, alcoyle en 1 à 4 atomes de carbone, alcoxy en 1 à 4 atomes de carbone ou trifluorométhyle.

2. Composé suivant la revendication 1, dans lequel R² est phényle, méthoxyphényle, chlorophényle, 3-trifluorométhylphényle, pyridinyle ou pyrimidinyle.

3. Composé suivant la revendication 1 ou 2, dans lequel m est 2 ou 3.

4. Composé suivant la revendication 1, dans lequel R¹ est 1-adamantyle, 3-méthyl-1-adamantyle, 2-indolyle, 2-benzofurannyle; R² est phényle substitué ou non substitué, où les substituants sont choisis parmi méthoxy et chloro, pyridinyle ou 2-pyrimidinyle; R³ est H; n est l'entier 0 ou 1; m est l'entier 2 ou 3, ou un sel pharmaceutiquement acceptable de celui-ci.

5. Composé suivant la revendication 1, qui est l'un des suivants :
le N-[3-[4-(2-méthoxyphényl)-1-pipérazinyl]propyl]tricyclo[3.3.1.1^{3,7}]décane-1-carboxamide;
le N-[2-[4-(2-pyrimidyl)-1-pipérazinyl]éthyl]tricyclo[3.3.1.1^{3,7}]décane-1-carboxamide;
le N-[2-[4-(2-méthoxyphényl)-1-pipérazinyl]éthyl]tricyclo[3.3.1.1^{3,7}]décane-1-carboxamide;
le N-[2-[4-(3-chlorophényl)-1-pipérazinyl]éthyl]tricyclo[3.3.1.1^{3,7}]décane-1-carboxamide;
le N-[2-[4-(2-pyrimidyl)-1-pipérazinyl]éthylbenzofuranne-2-carboxamide;
le N-[3-[4-(2-méthoxyphényl-1-pipérazinyl]propyl](1H)-indole-2-carboxamide;
le N-[3-[4-(3-chlorophényl)-1-pipérazinyl]propyl]tricyclo[3.3.1.1^{3,7}]décane-1-carboxamide;
le carboxamide de l'acide N-[2-[4-(2-pyrimidyl)-1-pipérazinyl]éthyl]-3-méthyltricyclo[3.3.1.1^{3,7}]décane-1-acétique;
le N-[3-[4-(2-pyrimidyl)-1-pipérazinyl]propyl]tricyclo[3.3.1.1^{3,7}]décane-1-carboxamide;
le carboxamide de l'acide N-[2-[4-(3-chlorophényl)-1-pipérazinyl]éthyl]-3-méthyltricyclo[3.3.1.1^{3,7}]décane-1-acétique;
le carboxamide de l'acide N-[2-[4-(2-méthoxyphényl)-1-pipérazinyl]éthyl]-3-méthyltricyclo[3.3.1.1^{3,7}]décane-1-acétique;
le N-[2-[4-(2-pyrimidyl)-1-pipérazinyl]éthyl]hexahydro-2,5-méthanopentalène-3a(1H)-carboxamide;
le N-[2-[4-(2-méthoxyphényl)-1-pipérazinyl]éthyl]hexahydro-2,5-méthanopentalène-3a(1H)-carboxamide;
le N-[2-[4-(3-chlorophényl)-1-pipérazinyl]éthyl]hexahydro-2,5-méthanopentalène-3a(1H)-carboxamide;
le N-[2-[4-(3-trifluorométhylphényl)-1-pipérazinyl]éthyl]hexahydro-2,5-méthanopentalène-3a(1H)-carboxamide;
le N-[3-[4-(2-pyrimidyl)-1-pipérazinyl]propyl]hexahydro-2,5-méthanopentalène-3a(1H)-carboxamide, ou
le N-[3-[4-(2-méthoxyphényl)-1-pipérazinyl]propyl]hexahydro-2,5-méthanopentalène-3a(1H)-carboxamide, ou un sel pharmaceutiquement acceptable de ceux-ci.

6. Composition pharmaceutique qui comprend un composé de formule (I), ou un sel pharmaceutiquement acceptable de celui-ci tel que défini à la revendication 1, en association avec un support pharmaceutiquement acceptable.

7. Procédé de préparation d'un composé de formule (I) tel que défini à la revendication 1 ou d'un sel pharmaceutiquement acceptable de celui-ci, qui comprend l'une des étapes suivantes :
(a) l'acylation d'un composé de formule (III) : 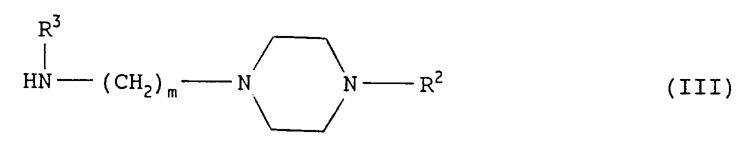 avec un agent acylant contenant le radical R⁵, dans lequel le radical R⁵ est :
R¹―(CH₂)ₙ―C(O)― (IIa)
où n, m, R¹, R², R³ sont tels que définis à la revendication 1, pour donner un composé de formule (I),
ou
(b) une amine cyclique de formule (IV) : 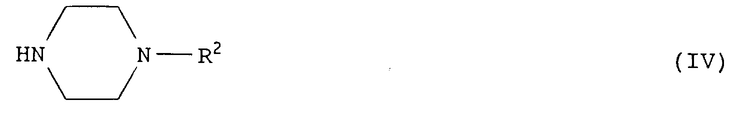 où R² est tel que défini à la revendication 1 ou un sel de celle-ci est alcoylé pour introduire le radical alcoyle substitué de formule (V) : 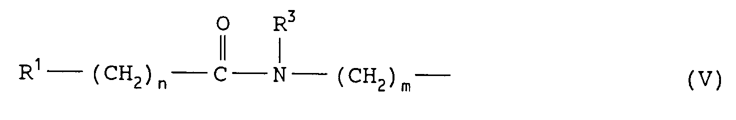 par réaction avec un composé ayant la formule R⁴-Y, où R⁴ est le radical ayant la formule (V) et Y est un groupe partant,
ou
(c) une amine cyclique ayant la formule (IV) telle que définie ci-dessus ou un sel de celle-ci est soumis à une alcoylation réductrice avec un aldéhyde de formule (VI) : 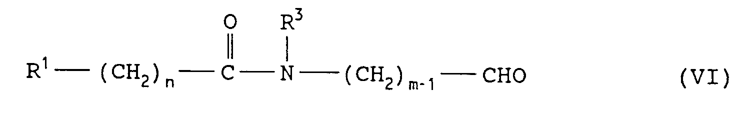 où n, R¹, R³ et m sont tels que définis à la revendication 1, ou
(d) un composé de formule (I) est converti en un sel d'addition d'acide de celui-ci par addition d'un acide ou un sel d'addition d'acide d'un composé ayant la formule (I) est soumis à neutralisation pour former le composé ayant la formule (I).

8. Procédé de préparation d'un composé ayant la formule (I) tel que défini à la revendication 1, ou d'un sel pharmaceutiquement acceptable de celui-ci, qui comprend la réaction d'un halogénure d'acide carboxylique de formule (II) : 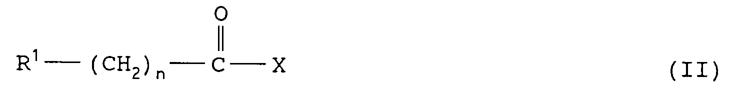 où R¹ et n sont tels que définis à la revendication 1 et X est un halogénure, avec une aminoalcoylpipérazine de formule (III) : 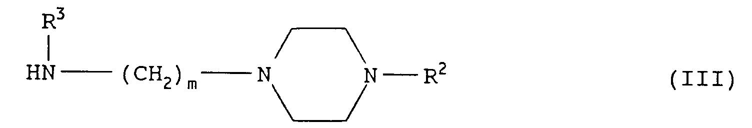 où R², R³ et m sont tels que définis ci-dessus.

9. Composé de formule (I) suivant la revendication 1, à utiliser comme produit pharmaceutique.

10. Utilisation d'un composé de formule : 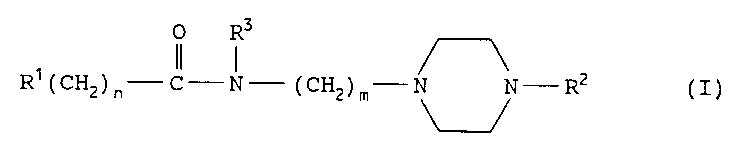 où :
R¹ est 1-adamantyle, 3-méthyl-1-adamantyle, 3-noradamantyle, 2-indolyle, 3-indolyle, 2-benzofurannyle ou 3-benzofurannyle substitué ou non substitué, où les substituants sont choisis parmi alcoyle inférieur en 1 à 6 atomes de carbone, alcoxy inférieur en 1 à 6 atomes de carbone et halo;
R² est phényle, benzyle, pyridinyle, pyrimidinyle ou pyrazinyle substitué ou non substitué, où les substituants sont choisis parmi alcoyle inférieur en 1 à 6 atomes de carbone, alcoxy inférieur en 1 à 6 atomes de carbone, trifluorométhyle et halo;
R³ est H ou alcoyle inférieur en 1 à 3 atomes de carbone;
n est l'entier de 0 ou 1, et
m est un entier de 2 à 5, ou un sel pharmaceutiquement acceptable de celui-ci, dans la préparation d'un médicament pour le traitement de l'anxiété, de la dépression ou de psychoses.

11. Utilisation suivant la revendication 10, dans laquelle le composé de formule (I) est le N-[3-[4-(2-méthoxyphényl)-1-pipérazinyl]propyl]benzofuranne-2-carboxamide ou un sel pharmaceutiquement acceptable de celui-ci.

## Revendications (Revendications pour l'(les) Etat(s) contractant(s) suivant(s): ES, GR)

1. Procédé de préparation d'un 5 composé de formule : 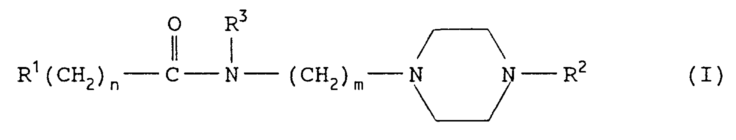 où :
R¹ est 1-adamantyle, 3-méthyl-1-adamantyle, 3-noradamantyle, 2-indolyle, 3-indolyle, 2-benzofurannyle ou 3-benzofurannyle substitué ou non substitué, où les substituants sont choisis parmi alcoyle inférieur en 1 à 6 atomes de carbone, alcoxy inférieur en 1 à 6 atomes de carbone et halo;
R² est phényle, benzyle, pyridinyle, pyrimidinyle ou pyrazinyle substitué ou non substitué, où les substituants sont choisis parmi alcoyle inférieur en 1 à 6 atomes de carbone, alcoxy inférieur en 1 à 6 atomes de carbone, trifluorométhyle et halo;
R³ est H ou alcoyle inférieur en 1 à 3 atomes de carbone;
n est l'entier 0 ou 1, et
m est un entier de 2 à 5, ou
un sel pharmaceutiquement acceptable de celui-ci, à la condition que, lorsque R¹ est 2-benzofurannyle substitué ou non substitué et lorsque n est 0, alors R² est autre que phényle ou phényle substitué par halogène, alcoyle en 1 à 4 atomes de carbone, alcoxy en 1 à 4 atomes de carbone ou trifluorométhyle, qui comprend l'une des étapes suivantes :
(a) l'acylation d'un composé de formule (III) : 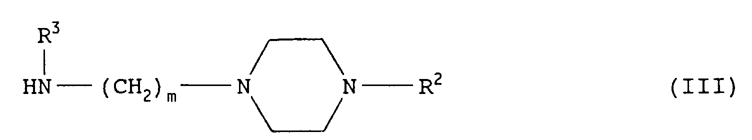 avec un agent acylant contenant le radical R⁵, dans lequel le radical R⁵ est :
R¹―(CH₂)ₙ―C(O)― (IIa)
où n, m, R¹, R², R³ sont tels que définis ci-dessus, pour donner un composé de formule (I),
ou
(b) une amine cyclique de formule (IV) : 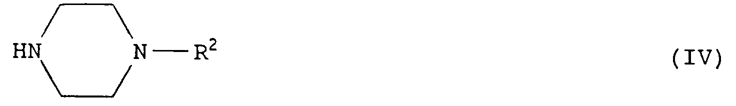 où R² est tel que défini à ci-dessus ou un sel de celle-ci est alcoylé pour introduire le radical alcoyle substitué de formule (V) : 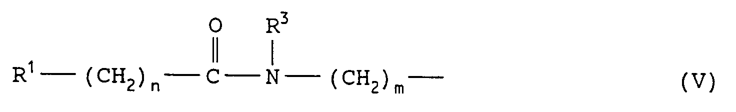 par réaction avec un composé ayant la formule R⁴-Y, où R⁴ est le radical ayant la formule (V) et Y est un groupe partant,
ou
(c) une amine cyclique ayant la formule (IV) telle que définie ci-dessus ou un sel de celle-ci est soumis à une alcoylation réductrice avec un aldéhyde de formule (VI) : 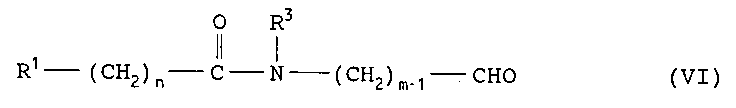 où n, R¹, R³ et m sont tels que définis ci-dessus,
ou
(d) un composé de formule (I) est converti en un sel d'addition d'acide de celui-ci par addition d'un acide ou un sel d'addition d'acide d'un composé ayant la formule (I) est soumis à neutralisation pour former le composé ayant la formule (I).

2. Procédé de préparation d'un composé ayant la formule (I) tel que défini à la revendication 1, ou d'un sel pharmaceutiquement acceptable de celui-ci, qui comprend la réaction d'un halogénure d'acide carboxylique de formule (II) : 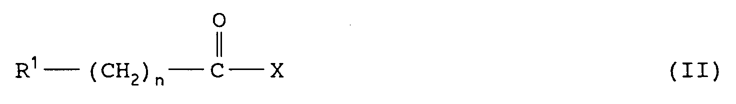 où R¹ et n sont tels que définis ci-dessus et X est un halogénure, avec une aminoalcoylpipérazine de formule (III) : 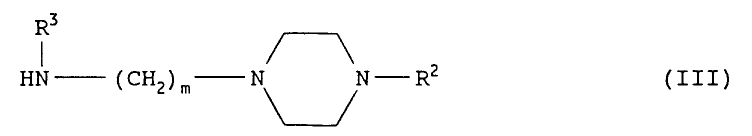 où R², R³ et m sont tels que définis ci-dessus.

3. Procédé suivant la revendication 1 ou 2, dans lequel R² est phényle, méthoxyphényle, chlorophényle, 3-trifluorométhylphényle, pyridinyle ou pyrimidinyle.

4. Procédé suivant l'une quelconque des revendications 1 à 3, dans lequel m est 2 ou 3.

5. Procédé suivant la revendication 1 ou 2, dans lequel R¹ est 1-adamantyle, 3-méthyl-1-adamantyle, 2-indolyle, 2-benzofurannyle; R² est phényle substitué ou non substitué, où les substituants sont choisis parmi méthoxy et chloro, pyridinyle ou 2-pyrimidinyle; R³ est H; n est l'entier 0 ou 1; m est l'entier 2 ou 3, ou un sel pharmaceutiquement acceptable de ceux-ci.

6. Procédé suivant la revendication 1 ou 2, dans lequel le produit est :
le N-[3-[4-(2-méthoxyphényl)-1-pipérazinyl]propyl]tricyclo[3.3.1.1^{3,7}]décane-1-carboxamide;
le N-[2-[4-(2-pyrimidyl)-1-pipérazinyl]éthyl]tricyclo[3.3.1.1^{3,7}]décane-1-carboxamide;
le N-[2-[4-(2-méthoxyphényl)-1-pipérazinyl]éthyl]tricyclo[3.3.1.1^{3,7}]décane-1-carboxamide;
le N-[2-[4-(3-chlorophényl)-1-pipérazinyl]éthyl]tricyclo[3.3.1.1^{3,7}]décane-1-carboxamide;
le N-[2-[4-(2-pyrimidyl)-1-pipérazinyl]éthylbenzofuranne-2-carboxamide;
le N-[3-[4-(2-méthoxyphényl-1-pipérazinyl]propyl])(1H)-indole-2-carboxamide;
le N-[3-[4-(3-chlorophényl)-1-pipérazinyl]propyl]tricyclo[3.3.1.1^{3,7}]décane-1-carboxamide;
le carboxamide de l'acide N-[2-[4-(2-pyrimidyl)-1-pipérazinyl]éthyl]-3-méthyltricyclo[3.3.1.1^{3,7}]décane-1-acétique;
le N-[3-[4-(2-pyrimidyl)-1-pipérazinyl]propyl]tricyclo[3.3.1.1^{3,7}]décane-1-carboxamide;
le carboxamide de l'acide N-[2-[4-(3-chlorophényl)-1-pipérazinyl]éthyl]-3-méthyltricyclo[3.3.1.1^{3,7}]décane-1-acétique;
le carboxamide de l'acide N-[2-[4-(2-méthoxyphényl)-1-pipérazinyl]éthyl]-3-méthyltricyclo[3.3.1.1^{3,7}]décane-1-acétique;
le N-[2-[4-(2-pyrimidyl)-1-pipérazinyl]éthyl]hexahydro-2,5-méthanopentalène-3a(1H)-carboxamide;
le N-[2-[4-(2-méthoxyphényl)-1-pipérazinyl]éthyl]hexahydro-2,5-méthanopentalène-3a(1H)-carboxamide;
le N-[2-[4-(3-chlorophényl)-1-pipérazinyl]éthyl]hexahydro-2,5-méthanopentalène-3a(1H)-carboxamide;
le N-[2-[4-(3-trifluorométhylphényl)-1-pipérazinyl]éthyl]hexahydro-2,5-méthanopentalène-3a(1H)-carboxamide;
le N-[3-[4-(2-pyrimidyl)-1-pipérazinyl]propyl]hexahydro-2,5-méthanopentalène-3a(1H)-carboxamide, ou
le N-[3-[4-(2-méthoxyphényl)-1-pipérazinyl]propyl]hexahydro-2,5-méthanopentalène-3a(1H)-carboxamide, ou un sel pharmaceutiquement acceptable de ceux-ci.

7. Procédé de préparation d'une composition pharmaceutique qui comprend la mise en association d'un composé de formule (I), ou d'un sel pharmaceutiquement acceptable de celui-ci, tel que défini à la revendication 1, avec un support pharmaceutiquement acceptable.

8. Utilisation d'un composé de formule : 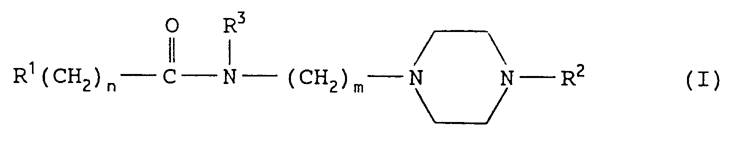 où :
R¹ est 1-adamantyle, 3-méthyl-1-adamantyle, 3-noradamantyle, 2-indolyle, 3-indolyle, 2-benzofurannyle ou 3-benzofurannyle substitué ou non substitué, où les substituants sont choisis parmi alcoyle inférieur en 1 à 6 atomes de carbone, alcoxy inférieur en 1 à 6 atomes de carbone et halo;
R² est phényle, benzyle, pyridinyle, pyrimidinyle ou pyrazinyle substitué ou non substitué, où les substituants sont choisis parmi alcoyle inférieur en 1 à 6 atomes de carbone, alcoxy inférieur en 1 à 6 atomes de carbone, trifluorométhyle et halo;
R³ est H ou alcoyle inférieur en 1 à 3 atomes de carbone;
n est l'entier 0 ou 1, et
m est un entier de 2 à 5, ou un sel pharmaceutiquement acceptable de celui-ci, dans la préparation d'un médicament pour le traitement de l'anxiété, de la dépression ou de psychoses.

9. Utilisation suivant la revendication 8, dans laquelle le composé de formule (I) est le N-[3-[4-(2-méthoxyphényl)-1-pipérazinyl]propyl]benzofuranne-2-carboxamide ou un sel pharmaceutiquement acceptable de celui-ci.
